# FASCICULE DE BREVET EUROPEEN

(11) **EP 0 495 902 B1**
(45) Date de publication et mention de la délivrance du brevet: **03.08.1994**
(21) Numéro de dépôt: 90916141.6
(22) Date de dépôt: 16.10.1990
(51) Int. Cl.: B65D 88/22, A61F 5/04, B63B 35/28, B63B 35/79, B63B 3/13

(54) **COQUE RIGIDE ENVELOPPANTE, DELIMITANT UN VOLUME INTERNE ETANCHE**
STARRE UMHÜLLENDE SCHALE MIT DICHTEM INNENVOLUMEN
RIGID WRAPAROUND SHELL DEFINING A SEALED INNER SPACE

(30) Priorité: 16.10.1989 FR 8913479
(43) Date de publication de la demande: 29.07.1992
(73) Titulaire: CONSTRUCTIONS INDUSTRIELLES DE LA MEDITERRANEE (CNIM), F-75008 Paris (FR)
(72) Inventeur: LEPINOY, Dominique, F-21000 Dijon (FR)
(74) Mandataire: Schrimpf, Robert
(86) Numéro de dépôt international: FR9000743
(87) Numéro de publication internationale: WO9105719

(56) Documents cités:
- FR-A- 1 270 443
- FR-A- 2 299 874
- FR-A- 2 377 931
- GB-A- 1 095 311
- US-A- 3 745 998
- US-A- 4 493 877

## Description

La présente invention concerne un dispositif du type comportant une coque rigide enveloppante, délimitant un volume interne, et au moins une chambre étanche intérieure occupant ledit volume interne.

Les dispositifs de ce type sont nombreux, et peuvent ressortir de domaines techniques variés.

On peut citer par exemple certains réservoirs, dont la chambre étanche intérieure est destinée à recevoir un fluide que l'on désire stocker pour être ensuite vidée de ce fluide, ou encore certains dispositifs flottants ou glissants tels que des flotteurs notamment de bâteaux, des coques de bâteau, des planches à voile, des luges ou des skis dont la chambre étanche intérieure peut contenir un gaz ou un matériau de remplissage présentant généralement une densité relative inférieure à 1, comme par exemple une matière alvéolaire.

Traditionnellement, la coque rigide de tels dispositifs est réalisée en métal ou en matière thermoplastique ou thermodurcissable et éventuellement, notamment dans le cas de dispositifs flottants, en bois si bien qu'elle présente un poids élevé ; en outre, du fait de la rigidité de cette coque, le dispositif qui en est muni présente une conformation et un encombrement constants, rendant malaisés sa manipulation et son stockage lorsqu'il n'est pas en service.

Pour remédier à ces inconvénients, on a proposé de remplacer la coque rigide de certains de ces dispositifs connus, et notamment des flotteurs et des réservoirs, par une membrane souple enveloppant la chambre étanche intérieure dont seul un remplissage en fluide en forte surpression relative, c'est-à-dire en forte surpression par rapport à la pression régnant à l'extérieur de cette chambre étanche, communique à la coque une forme définie et son encombrement maximal, par gonflage.

Cette technique peut certes autoriser un allègement, dans le cas de certains dispositifs comme par exemple les flotteurs de bâteau, mais la nécessité de mettre la chambre étanche intérieure en forte surpression rend la mise en service du dispositif particulièrement longue et pénible, à moins de disposer d'un compresseur lui-même lourd et encombrant, et en outre tributaire d'une source d'énergie.

Le but de la présente invention est de remédier à ces inconvénients, en proposant un nouveau mode de réalisation d'un dispositif du type indiqué en préambule ; un autre but de la présente invention est d'augmenter les possibilités d'application d'un dispositif de ce type, en en prévoyant notamment des modes de réalisation spécialement adaptés au calage et/ou soutien du corps humain dans une posture naturelle ou avec mise en posture prédéterminée, par exemple du type matelas ou coussin anti-escarres ou matelas chirurgical, ou encore au calage et/ou soutien d'au moins un objet dans une cavité présentant un contour interne, telle qu'un bagage, un conteneur ou un coffre de véhicule.

A cet effet, le dispositif selon l'invention est du type comportant une coque rigide enveloppante, délimitant un volume interne et constituée de parois étanches, souples, délimitant entre elles une enceinte étanche placée en dépression par rapport à une pression ambiante, et d'un matériau de remplissage emplissant intégralement ladite enceinte étanche placée en dépression, ledit matériau de remplissage étant d'un type se présentant à l'état rigide dans ladite enceinte étanche placée en dépression et susceptible de se présenter à l'état souple lorsqu'il est placé à la pression ambiante.

Un dispositif de ce type est décrit dans US-A-3 745 998 en référence à des applications à la contention, sous la forme d'attelles ou de matelas dits "coquilles". Dans de telles applications, la coque est destinée à épouser étroitement un membre ou une autre zone, plus ou moins importante, du corps d'un patient, et son volume interne est intégralement occupé par ce membre ou cette zone.

Le dispositif selon l'invention, bien que de ce type, se caractérise quant à lui en ce qu'il comporte au moins une chambre étanche intérieure occupant ledit volume interne.

Ledit matériau de remplissage peut être par exemple un matériau granulaire retenu par un réseau perméable aux gaz ou aux liquides mais imperméable à ce matériau granulaire, afin d'assurer une répartition prédéterminée avant l'établissement de la dépression dans l'enceinte étanche, comme l'enseigne WO 87/06209 qui cite à titre d'exemples non limitatifs de matériau granulaire un concassé de liège, de la sciure, des micro-billes du type commercialisé sous la marque "Vermiculite" et des billes de polystyrène expansé, et à titre d'exemple de moyens permettant de réaliser un réseau perméable aux gaz et aux liquides mais imperméable au matériau granulaire un cloisonnement de préférence indépendant des parois de l'enceinte ; de tels matériaux granulaires et un tel mode de constitution d'un réseau de retenue de celui-ci peuvent être également utilisés pour la mise en oeuvre de la présente invention, étant entendu que d'autres types de matériaux de remplissage susceptibles de se présenter à l'état rigide lorsqu'ils sont enfermés dans une enceinte étanche délimitée par des parois souples et placée en dépression, et à l'état souple lorsqu'ils sont soumis à la pression ambiante pourraient être utilisés sans que l'on sorte pour autant du cadre de la présente invention, notamment en fonction de l'utilisation projetée pour le dispositif selon l'invention, qui peut impliquer certains choix en fonction de la plus ou moins grande rigidité souhaitée pour la coque.

Un Homme du métier comprendra cependant aisément que des matériaux de remplissage légers pourront être utilisés tout en communiquant à la coque, du fait de la mise en dépression de l'enceinte étanche dans laquelle ils se trouvent, une rigidité convenable pour des applications à la réalisation de dispositifs flottants et/ou glissants choisis dans un groupe comportant les flotteurs, coques de bâteau, planches à voile, luges, skis, dont une certaine rigidité constitue un facteur de sécurité pour l'utilisateur et, souvent, une condition impérative de fonctionnement, ou à la réalisation de réservoirs, même pour des fluides craignant les chocs, ou encore à la réalisation de dispositifs de calage et/ou de soutien du corps humain, dans une posture naturelle ou avec mise en posture prédéterminée, choisis dans un groupe comportant les matelas ou coussins anti-escarres et les matelas chirurgicaux, ou de dispositifs de calage et/ou de soutien d'au moins un objet dans une cavité telle qu'un bagage, un conteneur, un coffre de véhicule ou analogue ; dans ces applications, à la possibilité d'obtenir une coque rigide s'ajoute la possibilité de conformer cette coque rigide en fonction des besoins, du fait de la possibilité de placer provisoirement l'enceinte étanche à la pression ambiante pour que le matériau de remplissage présente une souplesse permettant de modeler la coque ; cette possibilité peut également être utilisée pour réduire l'encombrement du dispositif selon l'invention lorsqu'il n'est pas utilisé, comme on peut le faire avec un canot pneumatique ou un matelas pneumatique, dont le gonflage ultérieur, nécessairement en forte surpression par rapport à la pression ambiante, constitue cependant une opération longue et fastidieuse alors que le retour d'un dispositif selon l'invention à sa conformation d'origine peut s'effectuer par un remplissage de la chambre étanche intérieure en un matériau fluide tel qu'un gaz ou mélange gazeux en faible surpression par rapport à la pression ambiante, pour rendre à la coque sa conformation d'origine, puis mise en dépression de l'enceinte étanche, interne à la coque, pour rigidifier cette dernière, ce qui oblige à un travail moindre.

Naturellement, en fonction des applications envisagées, un dispositif selon l'invention peut présenter de nombreuses dispositions annexes, en combinaison ou isolément.

Notamment, des modes de mise en oeuvre particuliers du dispositif selon l'invention se caractérisent en ce que :
- il comporte des moyens formant valve d'accès à l'intérieur de ladite enceinte étanche placée en dépression, ce qui permet d'une part de rétablir cette dépression au cas où elle viendrait à disparaître accidentellement, avec pour résultat un assouplissement indésirable de la coque rigide, et d'autre part d'intervenir volontairement sur cette dépression pour la faire disparaître à volonté en autorisant ou en provoquant l'introduction d'un gaz ou mélange gazeux, tel que de l'air, à la pression ambiante ou d'un liquide à la pression ambiante dans ladite enceinte étanche, afin de permettre un remodelage de la coque rigide, puis rétablir ensuite cette dépression, par extraction forcée de ce gaz, ou mélange gazeux, ou liquide, pour rigidifier à nouveau la coque ;
- la chambre étanche intérieure contient un matériau fluide choisi dans un groupe comportant les gaz, les gels, les liquides, les matériaux particulaires, généralement à une pression sensiblement identique à la pression ambiante bien que de préférence legerement supérieure à celle-ci, puisqu'il n'est pas indispensable de faire régner dans la chambre étanche intérieure une surpression importante dans la mesure où la préservation de la conformation de la coque est liée davantage à la rigidité de cette dernière qu'à la pression établie dans la chambre étanche intérieure ; cependant, dans certaines applications, ledit matériau fluide peut également être placé à une pression sensiblement supérieure à la pression ambiante ; ainsi, dans une application à la réalisation d'un réservoir de stockage de gaz ou mélange gazeux, le gaz ou mélange gazeux logé dans la chambre étanche intérieure peut être placé en surpression par rapport à la pression ambiante, la coque rigide présentant une rigidité suffisante pour s'opposer à l'expansion de la chambre étanche intérieure même dans un tel cas ; de même, dans une application à la réalisation d'un dispositif flottant ou glissant, le remplissage de la chambre étanche intérieure en un matériau fluide en surpression par rapport à la pression ambiante peut contribuer à la rigidité du dispositif en complément de l'effet de rigidification dû à la coque ; en comparaison avec des dispositifs connus, tels que des réservoirs ou des flotteurs, dont la chambre étanche intérieure est délimitée par une membrane souple, le remplacement de cette dernière par la coque rigidifiée par mise en dépression conformément à la présente invention autorise, à valeur égale de la surpression régnant dans la chambre étanche intérieure, un accroissement de la durée de vie des dispositifs en réduisant les contraintes résultant de ladite surpression dans les matériaux les constituant et dans les moyens d'assemblage de ces matériaux, c'est-à-dire les risques de déchirure, de rupture ou d'éclatement, ou permet, à durée de vie équivalente, d'augmenter ladite surpression ;
- le dispositif comporte des moyens pour faire circuler ledit matériau fluide ou, de façon plus générale, des moyens formant valve d'accès à l'intérieur de ladite chambre étanche intérieure ;
- le dispositif comporte ladite chambre étanche intérieure en plusieurs exemplaires occupant conjointement ledit volume interne, étant entendu que c'est chacun desdits exemplaires qui peut présenter les caractéristiques énumérées ci-dessus à propos de la chambre étanche intérieure ; ainsi, dans une application à un réservoir, il est possible de prévoir la chambre étanche intérieure en deux exemplaires au moins, et de prévoir en outre des moyens pour, en alternance, introduire un premier fluide à stocker dans un premier desdits exemplaires en vidant un second desdits exemplaires et introduire un deuxième fluide de commande dans ledit second exemplaire en chassant le fluide à stocker dudit premier exemplaire, ce qui permet de réaliser de façon particulièrement commode le remplissage et la vidange du réservoir ; dans une application au calage et/ou soutien du corps humain, par exemple sous la forme d'un matelas ou coussin anti-escarres ou d'un matelas chirurgical, application dans laquelle ladite coque rigide présente de préférence une zone supérieure de forme générale plate, une zone inférieure de forme générale plate, et une zone périphérique en forme de jupe reliant mutuellement lesdites zones supérieure et inférieure de formes générales respectives plates, on peut prévoir qu'au moins certains desdits exemplaires présentent la forme de plots mutuellement juxtaposés sous ladite zone supérieure et/ou encore qu'au moins certains desdits exemplaires présentent la forme de boudins de même direction longitudinale déterminée, mutuellement juxtaposés transversalement à ladite direction, sous ladite zone supérieure, que d'autres desdits exemplaires présentent la forme de boudins de même direction transversale, lesdits boudins de même direction longitudinale déterminée et lesdits boudins de même direction transversale étant mutuellement superposés sous ladite zone supérieure, et de prévoir en outre des moyens pour provoquer de façon commandée une succession d'étapes consistant à placer ladite enceinte étanche à la pression ambiante, gonfler ou dégonfler de façon déterminée certains, déterminés, desdits exemplaires, replacer ladite enceinte étanche en dépression par rapport à la pression ambiante ; de préférence, lesdites parois étanches délimitant l'enceinte étanche sont comparativement inextensibles dans ladite zone périphérique, dans laquelle elles jouent un rôle de contention, et comparativement élastiquement extensibles dans ladite zone supérieure dans laquelle elles se moulent ainsi au mieux sur l'utilisateur, lorsque l'enceinte étanche est à la pression ambiante ; dans le cas d'un matelas ou coussin anti-escarres, il est ainsi possible de faire varier le mode de répartition du poids du corps sur la zone supérieure de la coque rigide, en agissant soit manuellement à la demande, soit de façon automatique et commandée par un automate couplé par exemple à des capteurs de pression répartis sur la zone supérieure de la coque rigide et/ou à une minuterie ; le recours à un tel automate, dont la conception ressort du domaine des aptitudes normales d'un Homme du métier, permet d'assurer une gestion rigoureuse des positionnements d'un corps humain en lit ou en fauteuil de façon à étaler statistiquement dans la journée les zones d'appui du corps sur le matelas ou coussin, et les pressions d'appui dans ces zones d'appui en faisant intervenir non seulement les zones naturelles d'appui mais également des zones du corps qui, normalement, ne servent pas à l'appui de celui-ci comme par exemple l'arrière des genoux et en assurant une répartition du poids du corps sur une surface telle qu'il n'y ait pas douleur, ce qui permet d'accéder à un véritable traitement dynamique de l'escarre et à une véritable prévention dynamique de l'escarre ; il devient également facile de placer puis de caler, momentanément, l'utilisateur dans une posture désirée par exemple pour sa toilette ou pour un changement de draps, ou encore pour un examen ou pour des soins ; dans le cas d'un matelas chirurgical, il est possible de placer ainsi le corps d'un patient dans la posture la mieux adaptée à l'intervention chirurgicale à effectuer tout en assurant un calage optimal grâce à la rigidité que présente la coque dès lors que l'enceinte étanche qu'elle présente intérieurement est placée en dépression par rapport à la pression ambiante ; dans l'un et l'autre cas, le confort du matelas ou coussin reste optimal en dépit de la rigidité de la coque dans la mesure où celle-ci se moule directement sur le corps alors que ladite enceinte étanche se trouve à la pression ambiante, et ceci dans des conditions assurant une équirépartition des pressions d'appui en regard de chaque exemplaire de chambre étanche intérieure ou boudin ; dans de telles applications, il est également possible de faire circuler dans l'un au moins des exemplaires de chambre étanche intérieure un liquide ou un gel porté à une température déterminée pour, selon les besoins, réchauffer ou réfrigérer certaines parties du corps ;
- ladite chambre étanche intérieure, respectivement au moins l'un desdits exemplaires, est dissociable de ladite coque rigide, ce qui facilite la fabrication du dispositif ou sa réparation en cas d'avarie et, en outre, permet de remplacer une chambre étanche intérieure, respectivement un exemplaire de chambre étanche intérieure, destiné à contenir un matériau déterminé, par une chambre étanche intérieure ou un exemplaire de chambre étanche intérieure plus particulièrement adapté à contenir un autre matériau déterminé, en fonction des besoins notamment dans des applications à la réalisation de réservoirs ou de dispositifs de calage et/ou de soutien du corps humain.

De même, selon les applications auxquelles est destiné le dispositif selon l'invention, celui-ci peut présenter certaines des caractéristiques suivantes :
- il peut comporter une housse souple enveloppant ladite coque rigide à l'extérieur dudit volume interne, notamment dans le cas d'applications de la présente invention à la réalisation de coussins ou matelas anti-escarres ou de matelas chirurgicaux ; dans le cas d'une application au calage et/ou soutien du corps humain, cette housse peut avantageusement être comparativement inextensible dans une zone correspondant à ladite zone périphérique et comparativement élastiquement extensible dans une zone correspondant à ladite zone supérieure, respectivement pour jouer au mieux un rôle de contention et pour s'adapter au mieux à la forme de l'utilisateur lorsque l'enceinte étanche est à la pression ambiante ; elle peut en outre présenter toute disposition propre à augmenter le confort de contact pour l'utilisateur et notamment tout revêtement et/ou toute texture appropriée ; en particulier, elle peut se présenter notamment dans sa zone correspondant à ladite zone supérieure sous forme d'un matériau multicouche réalisé par tout procédé approprié, et par exemple par contre-collage thermique, en compression, de plusieurs couches de matériaux souples comparativement incompressibles entre lesquelles on insère une couche d'un matériau souple comparativement élastiquement compressible ;
- ladite coque rigide enveloppe intégralement, de façon continue, ledit volume interne, ce qui peut être le cas par exemple dans une application à un réservoir dont la coque rigide n'est pas appelée à connaître des chocs ou frictions susceptibles d'endommager ses parois étanches ;
- au contraire, ladite coque rigide présente des lèvres délimitant une discontinuité localisée de cette coque rigide, auquel cas lesdites lèvres sont soit mutuellement jointives, de telle sorte que la coque rigide enveloppe intégralement ledit volume interne, et des moyens d'assemblage mutuel desdites lèvres peuvent être prévus, soit mutuellement espacées, auquel cas ladite coque rigide enveloppe partiellement, de façon discontinue, ledit volume interne et des moyens de liaison mutuelle desdites lèvres mutuellement espacées peuvent être prévus ; ces moyens de liaison mutuelle peuvent se présenter sous plusieurs modes de réalisation pratiques selon la destination du dispositif selon l'invention ; ils peuvent être portés par lesdites lèvres ou être portés et/ou constitués par la housse souple précitée lorsqu'une telle housse est prévue et, selon les cas, peuvent être élastiquement déformables en extension et/ou en torsion et/ou en flexion entre lesdites lèvres, ou encore être élastiquement déformables en compression ; dans le cas d'une application à la réalisation d'un matelas ou coussin anti-escarres ou d'un matelas chirurgical, ces moyens de liaison peuvent comporter au moins un lien souple, déformable aussi bien en extension qu'en torsion et en flexion entre lesdites lèvres alors que dans une application à la réalisation d'un dispositif flottant et/ou glissant, ou d'un réservoir exposé aux chocs ou aux frottements de façon localisée, ils peuvent se présenter sous la forme d'une paroi complétant ladite coque rigide pour délimiter ledit volume interne de façon continue, en pratique sous forme d'une paroi inférieure du dispositif s'il s'agit d'un dispositif flottant et/ou glissant, ou encore de façon discontinue et par exemple sous forme d'une paroi annulaire délimitant un trou de passage pour un mât dans le cas d'un tel dispositif ; de tels moyens de liaison mutuelle des lèvres mutuellement espacées peuvent également être omis dans certaines applications, par exemple dans l'application à un dispositif de calage et/ou de soutien d'au moins un objet dans une cavité telle qu'un bagage, un conteneur, un coffre de véhicule ; la coque rigide elle-même peut être protégée à l'encontre des chocs et/ou des frottements par des moyens de protection appropriés, localisés ou non, accolés à la coque à l'extérieur dudit volume interne et portés par la coque elle-même ou encore portés et/ou constitués par la housse précitée lorsqu'une telle house est prévue ; ces moyens de protection peuvent comporter notamment au moins une paroi souple délimitant une chambre étanche extérieure placée en surpression par rapport à la pression ambiante, ou encore un coussin d'un matériau alvéolaire ; on peut prévoir de tels moyens de protection en guise de pare-battage dans le cas de l'application du dispositif selon l'invention en tant que flotteur ou coque de bâteau, auquel cas il s'agit de protéger le flotteur ou à la coque contre les chocs et/ou frottements latéraux mais, par exemple dans le cas de la réalisation d'une coque de bâteau, on peut prévoir de tels moyens pour protéger un utilisateur à l'encontre des chocs et/ou des frottements contre la coque rigide, par exemple en les localisant dans une zone de la coque rigide formant un siège pour l'utilisateur de même que dans le cas de la réalisation d'un bagage sous forme d'un sac à dos, de tels moyens peuvent être prévus pour protéger le dos d'un utilisateur à l'encontre des frottements ; dans le cas d'une application à la réalisation d'un matelas ou coussin anti-escarres ou d'un matelas chirurgical, on peut également prévoir de tels moyens pour augmenter le confort de contact avec l'utilisateur, notamment sous forme d'une chambre étanche extérieure accolée à la coque rigide à l'extérieur dudit volume interne et contenant un matériau fluide choisi dans un groupe comportant les gaz, les gels, les liquides, les matériaux particulaires, par exemple placé à une pression sensiblement identique ou encore sensiblement supérieure à la pression ambiante et que l'on peut si on le désire faire circuler par exemple pour établir une régulation thermique du corps de l'utilisateur ou grâce auquel on peut créer un effet de massage dans un mode de réalisation selon lequel ladite chambre extérieure est prévue en plusieurs exemplaires et des moyens sont prévus pour établir de façon prédéterminée, grâce à un automate, des pressions respectives prédéterminées, variables, dans chacun desdits exemplaires ; la chambre étanche extérieure ou chaque exemplaire de celle-ci peut être porté directement par la coque rigide elle-même ou être porté et/ou constitué par la housse souple éventuelle.

Bien que, dans de nombreuses applications du dispositif selon l'invention, la chambre étanche, éventuellement prévue en plusieurs exemplaires, occupe intégralement le volume interne de la coque rigide, on peut également prévoir en vue de certaines applications que le dispositif comporte des moyens définissant un relief déterminé à l'intérieur dudit volume interne, et de préférence amovible ; c'est par exemple le cas lorsqu'on réalise conformément à la présente invention un matelas chirurgical, dans lequel on peut ainsi insérer un billot sensiblement incompressible ou au contraire offrant une compressibilité choisie, connue, et par exemple un billot pneumatique ; lorsque, comme il est préféré dans le cas d'une telle application et comme on l'a indiqué plus haut, la coque rigide présente des lèvres mutuellement espacées délimitant une discontinuité localisée de cette coque rigide, notamment dans une zone inférieure de la coque, et que sont prévus des moyens de liaison mutuelle desdites lèvres mutuellement espacées, ces moyens de liaison mutuelle peuvent avantageusement présenter au moins une protubérance localisée à l'intérieur dudit volume interne, cette protubérance localisée pouvant être en contact avec une zone localisée de la coque rigide, en pratique une zone localisée supérieure de celle-ci, pour constituer un appui mutuel localisé entre la coque rigide et les moyens de liaison mutuelle, eux-mêmes supposés en appui sur un support tel qu'une table d'opération ; de façon plus générale, dans de nombreuses applications de la présente invention, on peut prévoir une ou plusieurs protubérances localisées à l'intérieur du volume interne de la coque rigide, soit pour communiquer à celle-ci une forme de base au cas, par exemple, où une avarie lui ferait perdre sa rigidité et entraînerait également une perte d'étanchéité de la chambre étanche intérieure ou de certains exemplaires de celle-ci, soit pour permettre de limiter les volumes de fluide à déplacer pour emplir ou vider cette chambre étanche lorsque sa fonction essentielle n'est pas une fonction de stockage, par exemple de telle sorte que ces volumes équilibrent approximativement les volumes de gaz ou mélange gazeux, ou liquide qu'il est nécessaire respectivement d'extraire de l'enceinte étanche pour la rigidifier ou de laisser pénétrer dans l'enceinte étanche pour lui faire perdre sa rigidité, ce qui permet d'effectuer par transfert de fluide entre la chambre étanche intérieure et l'enceinte étanche la mise en forme de celle-ci et sa rigidification, ou son remodelage ; ainsi, on peut réaliser selon l'invention un coussin dont une partie supérieure est constituée par ladite coque et une partie inférieure par une paroi rigide ou semi-rigide délimitant avec ladite coque un volume interne formant une chambre étanche gonflable, et prévoir sur cette paroi, à l'intérieur dudit volume interne, des creux et reliefs de forme anatomique préservant un certain confort d'utilisation dans le cas d'une telle avarie. Complémentairement ou en variante, la chambre étanche intérieure peut également contenir un matériau expansé la remplissant totalement ou partiellement, ce matériau pouvant être élastiquement compressible et par exemple prévu pour combler totalement la chambre étanche intérieure et ainsi contribuer à la conformation de la coque lorsque cette dernière se trouve à l'état souple, ou sensiblement incompressible et par exemple prévu pour combler partiellement la chambre étanche et limiter ainsi les quantités de fluide à transférer respectivement pour l'emplir ou la vider lorsque sa fonction essentielle n'est pas une fonction de stockage ou pour communiquer ainsi au dispositif une forme déterminée, de base, lorsque la coque se trouve à l'état souple et que la chambre étanche est ouverte à l'air libre, accidentellement ou volontairement.

D'autres caractéristiques et avantages d'un dispositif selon l'invention ressortiront de la description ci-dessous, relative à plusieurs exemples non limitatifs d'un tel dispositif, ainsi que des dessins annexés qui font partie intégrante de cette description.
- La figure 1 montre une vue, en perspective et coupe partielle, d'un réservoir réalisé conformément à la présente invention.
- La figure 2 montre une vue, à échelle agrandie, d'un détail repéré en II à la figure 1.
- La figure 3 montre une vue, analogue à celle de la figure 1, d'un flotteur réalisé conformément à la présente invention.
- La figure 4 montre une vue en perspective, avec coupe partielle, d'une coque de bâteau réalisée conformément à la présente invention.
- La figure 5 montre, en une vue analogue à celle de la figure 1, un matelas anti-escarres réalisé conformément à la présente invention.
- La figure 6 montre, en une vue analogue à celle de la figure 1, un matelas chirurgical réalisé conformément à la présente invention.
- La figure 7 montre, en une vue analogue à celle de la figure 1, un autre exemple de matelas anti-escarres réalisé conformément à la présente invention.
- La figure 8 montre une vue de cet autre exemple de matelas anti-escarres selon l'invention, en coupe par un plan constituant pour ce matelas un plan longitudinal de symétrie, repéré en VIII-VIII à la figure 7.
- La figure 9 montre, en une vue analogue à celle de la figure 1, un coussin de siège réalisé conformément à la présente invention.
- La figure 10 montre, en une vue analogue à celle de la figure 1, un bagage tel qu'un sac de voyage équipé d'un dispositif de calage et/ou de soutien d'objets réalisé conformément à la présente invention.
- La figure 11 montre, en une vue en coupe par un plan vertical médian, un sac à dos réalisé conformément à la présente invention.

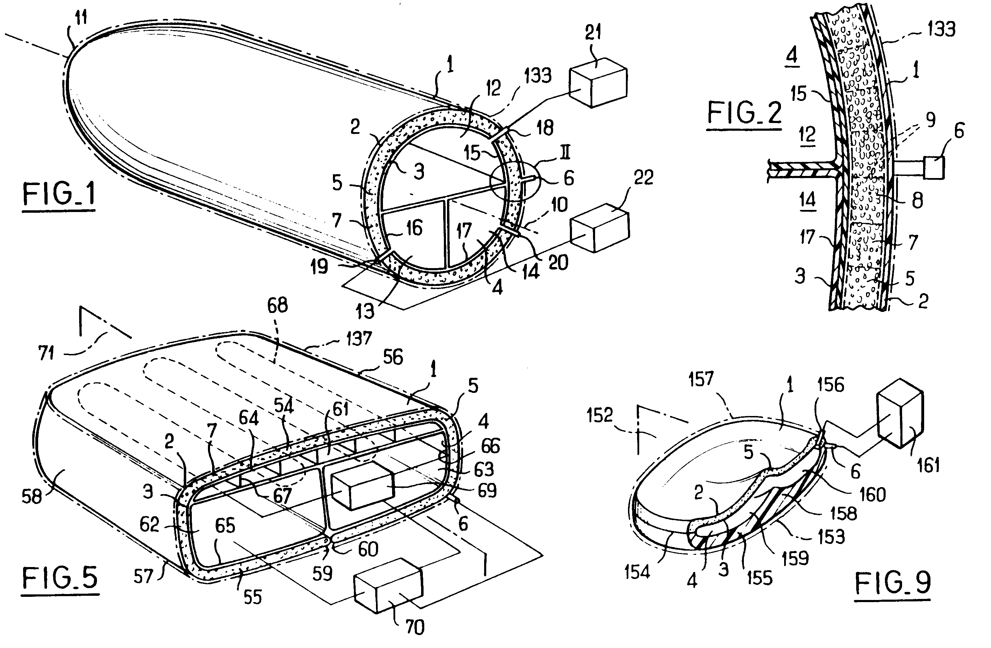

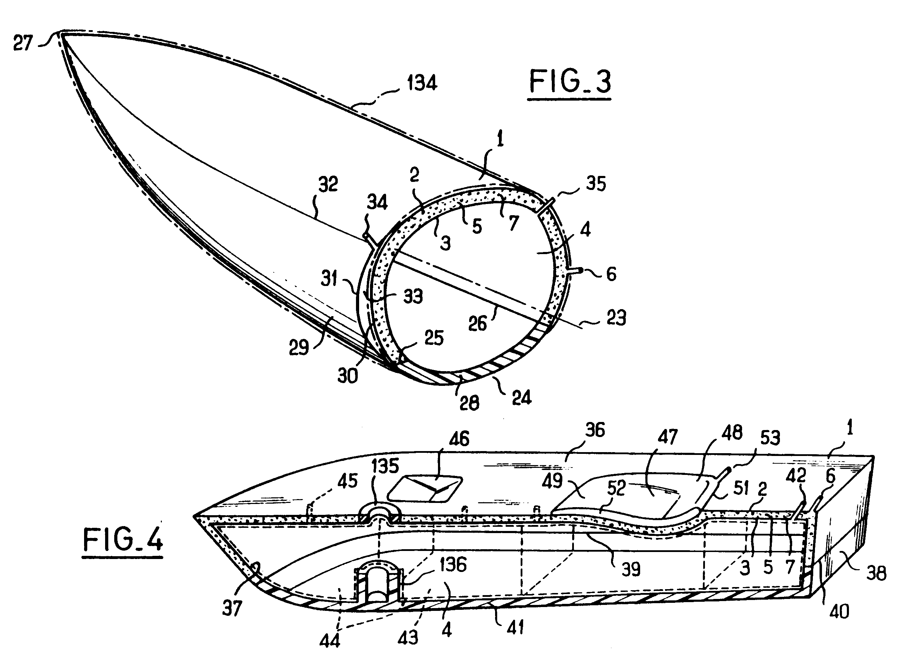

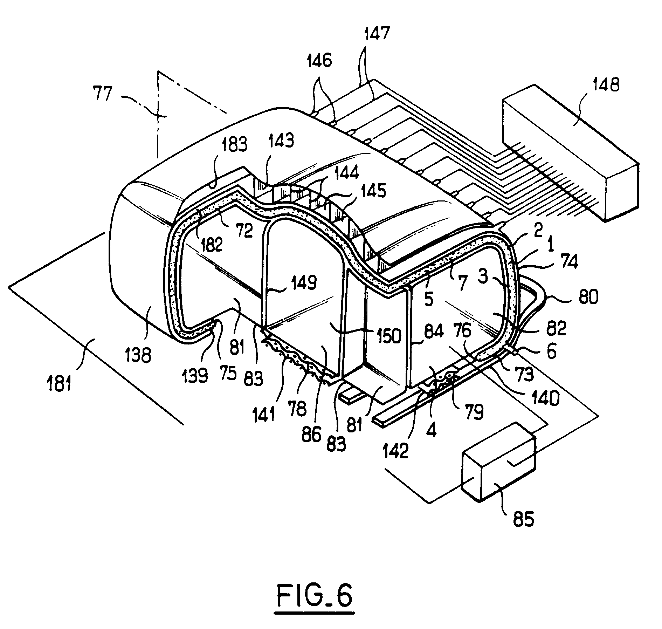

Sur l'ensemble de ces figures, on a désigné par 1 la coque rigide du dispositif selon l'invention, formée de deux parois souples, étanches 2, 3 tournées respectivement vers l'extérieur de la coque rigide 1 et vers l'intérieur de celle-ci, c'est-à-dire vers un volume interne 4 que la coque rigide enveloppe au moins pour l'essentiel (figures 3, 4, 6, 7, 8, 9, 10, 11), voire en totalité (figures 1, 5); les deux parois souples, étanches 2, 3 délimitent entre elles une enceinte étanche 5 placée en dépression par rapport à la pression ambiante, c'est-à-dire par rapport à la pression atmosphérique à laquelle la paroi 2 est soumise à l'extérieur de la coque rigide 1, et à la pression à laquelle la paroi 3 est soumise à l'intérieur de cette coque rigide 1 ; l'enceinte étanche 5, accessible par une valve 6 permettant d'établir ou de rétablir cette dépression ou, éventuellement, de placer l'enceinte étanche 5 à la pression ambiante, est intégralement emplie d'un matériau de remplissage 7 qui, de façon caractéristique, se présente à l'état rigide lorsqu'il est ainsi emprisonné entre les deux parois 2, 3 et que l'enceinte étanche 5 est placée en dépression relative, pour communiquer sa rigidité à la coque 1, alors qu'il se présente à l'état souple et communique une telle souplesse à la coque rigide 1 si l'enceinte étanche 5 est placée à la pression ambiante ; ce matériau de remplissage consiste par exemple, comme le montre la figure 2, en un matériau granulaire 8 qui peut être choisi parmi les matériaux indiqués dans WO 87/06209 précité ou encore être d'une autre nature, ce matériau granulaire 8 étant retenu à l'intérieur de l'enceinte étanche 5 par des moyens tels qu'un cloisonnement 9 formant un réseau étanche aux gaz ou aux liquides mais imperméable au matériau granulaire, comme l'enseigne WO 87/06209, de telle sorte que lorsque l'enceinte étanche 5 se trouve à la pression ambiante soit lors de la fabrication de la coque rigide 1, soit lorsqu'on désire ultérieurement modifier la forme de celle-ci, le matériau granulaire 8 conserve à l'intérieur de l'enceinte étanche 5 une répartition prédéterminée, c'est-à-dire n'aille pas s'accumuler dans certaines zones de l'enceinte étanche 5 au détriment d'autres zones de celles-ci ; ceci permet de conserver entre les deux parois 2 et 3 de la coque rigide 1 un écartement prédéterminé lorsque l'enceinte étanche 5 est mise en dépression ; dans l'ensemble des modes de mise en oeuvre de l'invention qui ont été illustrés, cet écartement apparait comme sensiblement constant, étant entendu que l'on ne sortirait pas du cadre de la présente invention en prévoyant un écartement différent suivant les zones de la coque rigide 1.

Selon l'application du dispositif selon l'invention, le volume interne 4 de la coque rigide 1 est au moins partiellement empli d'au moins une chambre étanche délimitée par des parois étanches de préférence indépendantes des parois 2 et 3 de la coque rigide 1, et de préférence mutuellement indépendantes lorsque cette chambre est prévue en plusieurs exemplaires, comme il ressortira de la description ci-après des différents modes de mise en oeuvre de l'invention illustrés aux figures 1 à 9.

On se réfèrera en premier lieu aux figures 1 et 2, au l'on a illustré la coque rigide 1 sous forme d'un boudin allongé, de révolution autour d'un axe longitudinal 10 et enveloppant intégralement, de façon continue, le volume interne 4 ; les parois 2 et 3 présentent dans ce cas pour l'essentiel une forme cylindrique de révolution autour de l'axe 10, sauf dans deux zones extrêmes telles que 11 dans lesquelles les parois 2 et 3 présentent des formes approximativement hémisphériques.

De préférence, comme on l'a illustré en trait mixte, une housse souple 133 enveloppe intégralement la coque rigide 1 par l'extérieur, c'est-à-dire au contact de la paroi 2, afin de protéger celle-ci contre les risques de déchirure ; cette housse 133 est de préférence élastiquement extensible, et placée en tension lorsque la coque 1 est à l'état rigide pour en épouser étroitement la paroi 2.

Intérieurement, le volume interne 4 de la coque rigide 1 est subdivisé en trois chambres longitudinales, étanches, 12, 13, 14 dont chacune est délimitée par ses propres parois souples, étanches, respectivement 15, 16, 17 et comporte sa propre valve d'accès 18, 19, 20 traversant de façon étanche les deux parois 2 et 3 de la coque rigide 1 ainsi que l'enceinte étanche 5 et le matériau de remplissage 7 logé dans celle-ci ; de préférence, les parois 15, 16, 17 sont élastiquement extensibles alors que, de façon générale, cette qualité n'est pas exigée des parois 2 et 3 de la coque rigide 1. De façon non représentée mais aisément concevable par un Homme du métier, on peut rendre les chambres 12, 13, 14 interchangeables en prévoyant dans une zone localisée de la coque 1 et de la housse 133 éventuelle une ouverture autour de laquelle les parois 2 et 3 sont solidarisées mutuellement de façon continue et étanche et qui permet d'extraire ou d'introduire à volonté une ou plusieurs des parois 15, 16, 17.

Les chambres 13 et 14 sont destinées à recevoir un fluide à stocker tel qu'un liquide ou un gaz, ou encore un matériau particulaire, par exemple des céréales, du riz ou d'autres matériaux en grains, alimentaires ou non, si les valves 19 et 20 sont remplacées par des dispositifs autorisant le passage d'un tel matériau, alors que la chambre 12 est destinée à recevoir un fluide de commande servant, à volonté, à créer un effet d'aspiration ou de refoulement du fluide à stocker dans les chambres 13 et 14.

Plus précisément, en raccordant la valve 18 à des moyens 21 d'aspiration et de refoulement, à volonté, d'un fluide de commande qui peut être de l'air, ou tout gaz, tout mélange gazeux ou tout liquide approprié, on peut créer un état initial du réservoir dans lequel la chambre 12 emplie de fluide de commande occupe intégralement le volume interne 4, les chambres 13 et 14 vides présentant un volume nul par écrasement total à l'intérieur de ce volume 4 ; si, ensuite, on raccorde les valves 19 et 20 à une source 22 de fluide à stocker, comme par exemple un réservoir de stockage intermédiaire, on peut en créant une aspiration dans la chambre 12 par les moyens 21 provoquer une réduction progressive du volume de cette chambre 12, accompagnée d'un accroissement progressif du volume des chambres 13 et 14 et ainsi créer un effet d'aspiration du fluide en provenance de la source 22, vers l'intérieur des chambres 13 et 14 qui s'emplissent progressivement de ce fluide ; la figure 1 illustre un état intermédiaire de cette vidange progressive de la chambre 12, accompagnée d'un remplissage concomitant des chambres 13 et 14 ; lorsque la chambre 12 a été totalement vidée du fluide de commande par les moyens 21, les chambres 13 et 14 occupent intégralement, conjointement, le volume interne 4 et le réservoir est plein ; les valves 18, 19 et 20 étant fermées et leur raccordement respectivement avec les moyens 21 et avec la source 22 étant supprimé, le réservoir plein peut être stocké et transporté ; on remarquera que lorsque le fluide à stocker est un liquide, le fait de prévoir en vue de sa réception plusieurs chambres, en pratique les chambres 13 et 14, permet d'éviter tout ballottement de ce liquide au cours du transport ; lorsque le fluide à stocker est un matériau particulaire, cet effet peut être renforcé si, après avoir empli les chambres 13 et 14 en ce matériau, on introduit par les moyens 21 du fluide de commande sous pression dans la chambre 12 et on tire au vide dans les chambres 13 et 14 par raccordement de celle-ci à une source de dépression substituée à la source 22 ; en outre, quelle que soit la nature du fluide à stocker, une telle subdivision accroît la sécurité d'utilisation du réservoir, comme il est connu de façon générale dans le domaine des réservoirs.

Lorsque, ensuite, on désire reprendre le fluide stocké dans les chambres 13 et 14, on peut si on le désire provoquer une expulsion forcée de ce fluide en ouvrant les valves 19 et 20, éventuellement raccordées à un dispositif utilisateur de ce fluide, et en raccordant à nouveau la valve 18 aux moyens 21, utilisés alors pour insuffler un fluide de commande sous pression dans la chambre 12, ce qui provoque l'expansion progressive de cette dernière jusqu'à occuper l'intégralité du volume interne 4, et par conséquent la réduction progressive du volume des chambres 13 et 14 ; si le fluide stocké dans les chambres 13 et 14 est d'un type propre à sortir naturellement des chambres 13 et 14, il suffit d'ouvrir la chambre 12 à l'air libre si le fluide de commande est de l'air, ou vers un réservoir de fluide de commande dans le cas d'une nature différente de celui-ci, et le remplissage de la chambre 12 s'effectue naturellement au fur et à mesure du vidage naturel des chambres 13 et 14.

Naturellement, au lieu d'être remplies simultanément puis vidées simultanément, les chambres 13 et 14 pourraient être remplies successivement, puis vidées successivement de la façon décrite.

Un Homme du métier comprendra aisément que le réservoir illustré aux figures 1 et 2 peut être utilisé non seulement pour le stockage provisoire d'un fluide, mais également dans des utilisations voisines, et par exemple à titre de ballast dans un batyscaphe.

Un Homme du métier comprendra également que le nombre des chambres étanches à l'intérieur du volume interne 4 pourrait être différent de 3 et que, notamment, on pourrait substituer à la chambre 12 ou aux chambres 13 et 14, avec la même fonction et le même raccordement aux moyens 20 ou 21 respectivement, le volume interne 4 lui-même délimité de façon étanche, ou encore prévoir à l'intérieur du volume interne 4 une chambre étanche unique emplissant intégralement ce volume et éventuellement délimitée par la paroi 3 de l'enceinte étanche 5 elle-même, avec une vanne de vidange et un évent, pour permettre son utilisation à la façon d'une citerne rigide traditionnelle.

On se réfèrera à présent à la figure 3, où l'on a illustré un flotteur réalisé conformément à la présente invention.

Ce flotteur présente la forme d'un fuseau allongé, d'axe longitudinal 23 disposé horizontalement dans une position d'utilisation dans laquelle le flotteur est illustré à la figure 3.

Dans le cas de ce mode de mise en oeuvre de l'invention, la coque rigide 1 enveloppe le volume interne 4 de façon continue de toute part, sauf vers le bas, c'est-à-dire dans une zone inférieure 24 du flotteur ; dans cette zone inférieure 24, la coque rigide 1 présente deux lèvres longitudinales 25, 26 mutuellement espacées sur la majeure partie de la dimension longitudinale du flotteur et se raccordant mutuellement dans deux zones extrêmes telles que 27 de celui-ci ; ces deux lèvres 25 et 26 sont raccordées mutuellement, de façon continue et étanche, par une paroi 28 étanche qui coopère ainsi avec la coque 1 pour enfermer de toute part, de façon étanche, le volume interne 4.

La paroi 28 peut être rigide mais elle est de préférence élastiquement déformable en compression, en flexion et en torsion ; toutefois, elle présente une résistance aux chocs, aux frottements et à la déchirure, supérieure à celle de la coque 1 dans la mesure où c'est elle qui vient au contact de l'eau et, éventuellement des hauts-fonds ; les lèvres 25 et 26 sont situées approximativement au niveau de la ligne de flottaison ; au-dessus de celle-ci, une housse 134 en tout point comparable à la housse 133 si ce n'est qu'elle présente elle-même au niveau des lèvres 25 et 26 deux lèvres par lesquelles elle est solidarisée de façon continue avec la coque 1 et avec la paroi 28 peut être prévue pour protéger à l'encontre des déchirures la paroi 2 de la coque 1, qu'elle épouse étroitement.

Le flotteur illustré à la figure 3 comporte en outre un pare-battage intégré 29, localisé dans une zone longitudinale de flanc 30 de la coque 1, à l'extérieur du volume interne 4 de celle-ci. Ce pare-battage 29 présente la forme d'une paroi souple, étanche 31 présentant une périphérie 32 par laquelle elle est solidarisée de façon étanche avec la paroi 2 de la coque 1 ou avec la housse 134 de façon à délimiter avec cette paroi 2 ou avec la housse 134, respectivement une chambre étanche 33 accessible par une valve 34 permettant d'y introduire un fluide sous pression tel que de l'air sous pression, de façon à gonfler la chambre 33 ; à cet effet, la membrane 31 peut être choisie inextensible, mais suffisamment lâche à l'intérieur de sa périphérie 32 pour autoriser le gonflage de la chambre 33, ou encore être choisie d'une nature élastiquement extensible. D'autres modes de réalisation d'un tel pare-battage pourraient être prévus, par exemple sous la forme d'un coussin d'un matériau alvéolaire, élastiquement compressible, accolé à la coque 1 ou à la housse 134 et solidarise avec la coque 1 ou la housse 134, respectivement.

On remarquera que la paroi 28 de la zone inférieure 24 du flotteur complète la paroi 3 de la coque rigide 1 pour délimiter de façon étanche le volume interne 4 de celle-ci, si bien que ce volume interne 4 constitue lui-même une chambre étanche accessible par une valve 35 permettant d'y introduire par exemple un gaz ou mélange gazeux tel que de l'air à une pression proche de la pression ambiante, c'est-à-dire de la pression atmosphérique, et de préférence en légère surpression, sans que les valeurs de cette surpression atteignent les valeurs qu'il est nécessaire d'atteindre dans le cas de flotteurs gonflables, ou encore en surpression sensible par rapport à la pression ambiante pour faire contribuer le remplissage gazeux du volume interne 4 à la rigidité d'ensemble du flotteur ; on peut également prévoir un remplissage du volume interne 4 au moyen d'un matériau particulaire de densité relative inférieure à 1, ou d'un matériau expansé, étant entendu que ce choix peut être effectué indépendamment de tout souci de rigidifier le flotteur dans son ensemble, dont la rigidité peut résulter exclusivement de celle de la coque 1, résultant elle-même de la compression du matériau de remplissage 7 entre les parois 2 et 3 de l'enceinte étanche 5 placée en dépression relative.

Dans des variantes de réalisation du flotteur illustré à la figure 3, on pourrait cependant prévoir que le volume interne 4 de la coque rigide 1 soit compartimenté de façon étanche, par des parois y délimitant des chambres étanches à la façon dont les parois 15, 16, 17 délimitent des chambres étanches 12, 13, 14 dans le cas du réservoir illustré à la figure 1, afin notamment d'assurer une flottabilité au flotteur même en cas d'avarie grave, entraînant la perforation de la coque rigide 1 et la perte de rigidité de celle-ci par passage de l'enceinte étanche 5 à la pression ambiante.

Un Homme du métier comprendra aisément que la conception du flotteur illustré à la figure 3 peut être transposée à divers types de dispositifs flottants ou glissants, comme par exemple des planches à voile, des coques de bâteau et notamment de kayak, des luges ou des skis, dont la paroi 28, localisée dans une zone inférieure 24, constitue une semelle d'usure et sur lesquels des moyens analogues au pare-battage 29 peuvent constituer des protection des utilisateurs à l'encontre des chocs ; suivant les cas, la paroi 28 peut être choisie plus ou moins élastiquement déformable en torsion et/ou flexion et/ou en compression, en restant toutefois de préférence généralement inextensible entre les lèvres 25 et 26 de la coque 21.

La figure 4 illustre précisément une adaptation de la structure illustrée à la figure 3 au cas de la réalisation d'un bâteau, étant entendu qu'un Homme du métier pourra aisement en déduire une adaptation à la réalisation d'une luge.

On retrouve dans ce cas la coque 1, qui regroupe une zone supérieure de pont 36 de forme générale plate et horizontale si l'on se réfère à une position d'utilisation du bâteau, ainsi que des zones de flanc 37 et de tableau arrière 38 qui bordent de toute part la zone de pont 36 et se terminent vers le bas, au niveau de la ligne de flottaison, par des lèvres 39, 40 par lesquelles la coque 1 se solidarise de façon continue, étanche avec une paroi de fond 41 en tout point comparable à la paroi 28 du flotteur illustré à la figure 3 ; cette paroi de fond 41 délimite de façon étanche le volume interne 4 de la coque 1, en coopérant à cet effet avec les zones de flanc 37, de tableau arrière 38 et la zone de pont 36 elles-mêmes continues et raccordées mutuellement de façon continue ; ce volume interne 4 peut ainsi constituer lui-même une chambre étanche, accessible par une valve 42 permettant d'y introduire un gaz ou mélange gazeux tel que de l'air à une pression proche de la pression atmosphérique, et de préférence en légère surpression, ou un gaz ou mélange gazeux tel que de l'air en surpression sensible par rapport à la pression atmosphérique, ou encore tout matériau de remplissage approprié, notamment particulaire ou expansé, présentant une densité relative inférieure a 1 ; comme on l'a dit à propos du flotteur illustré à la figure 3, le volume interne 4 peut cependant être subdivisé par des cloisons souples telles que 43 y définissant des chambres étanches telles que 44 dont chacune peut être munie d'une valve propre 45 permettant d'y introduire un tel gaz ou mélange gazeux ou autre matériau de remplissage de densité relative inférieure à 1.

De façon particulièrement avantageuse, la zone de pont 36 peut présenter des creux et/ou reliefs permettant d'améliorer l'assise d'un utilisateur et on a ainsi illustré à la figure 4 une zone en creux 46 formant cale-pied, étant entendu qu'une autre zone analogue est prévue de façon non visible à cette figure, dans une zone de proue du bâteau, et une zone en creux 47 formant siège dans une zone de poupe du bâteau ; dans cette zone en creux 47, la coque 1 porte de façon solidaire un coussin 48 réalisé à la façon du pare-battage 29 du flotteur illustré à la figure 3, sous forme d'une paroi souple 49 étanche, extensible ou non, présentant une périphérie 51 le long de laquelle elle est solidarisée de façon continue, étanche, avec la paroi 2 de la coque 1 de façon à délimiter avec cette paroi 2 une chambre étanche 52 accessible par une valve 53 en vue de son gonflage par introduction d'un gaz ou mélange gazeux approprié, en surpression par rapport à la pression ambiante, ou encore au moyen d'un gel ou d'un matériau alvéolaire élastiquement compressible améliorant le confort de la zone formant siège 47.

En outre, la zone de pont 36 peut présenter localement des éléments insérés autour desquels les parois 2 et 3 sont solidarisées mutuellement, de façon continue et étanche, comme par exemple une pièce annulaire, rigide 135 prévue entre les deux zones formant cale-pied telles que 46 et destinée à permettre la traversée de la coque 1 par un mât non représenté et le calage de ce mât, par ailleurs reçu dans une protubérance en forme de manchon 136 que la paroi 41 présente en saillie à l'intérieur du volume interne 4 ; si celui-ci est destiné à constituer lui-même une chambre étanche, il convient d'assurer alors une étanchéité entre la pièce 135 et la protubérance 136 ; si des chambres étanches 44 sont définies dans le volume 4 par des cloisons 43, il convient de disposer celles-ci de façon à dégager un passage pour le mât entre la pièce 135 et la protubérance 136.

Naturellement, de façon non représentée, le bâteau illustré à la figure 4 pourrait comporter en vue de la protection de la coque 1 une housse souple en tout point comparable à la housse 134 et susceptible de porter le coussin 48 précité ; cette housse présenterait une interruption localisée en regard de la pièce 135. En outre, dans une variante non représentée, la zone de pont 36 pourrait être définie en tout ou partie non pas par la coque 1 mais par une paroi de pont en tout point analogue à la paroi de fond 41, à laquelle elle serait raccordée par la coque 1, alors limitée aux zones de flanc 37 et de tableau arrière 38 ; le raccordement des parois de pont et de fond à la coque 1 ainsi limitée s'effectuerait comme on l'a décrit à propos du raccordement de la paroi de fond 41 à la coque 1 du bateau illustré à la figure 4 et la paroi définissant ainsi la zone de pont 36 pourrait présenter toutes les dispositions décrites en relation avec cette dernière, telles que des zones en creux formant cale-pied, un passage pour un mât et un coussin 48, de façon aisément compréhensible par un Homme du métier ; naturellement, une telle structure groupant plusieurs parois analogues à la paroi 41 du bateau illustré à la figure 4 et une coque analogue à la coque 1, ou plusieurs coques analogues à la coque 1, pour délimiter un volume interne 4 logeant au moins une chambre étanche pourrait se retrouver dans d'autres domaines d'application de la présente invention, et par exemple dans des applications à la réalisation de réservoirs ou de flotteurs, ces exemples n'étant nullement limitatifs.

On remarquera que, dans le cas de l'application de l'invention à la réalisation de dispositifs flottants tels que le flotteur illustré à la figure 3 ou le bâteau illustré à la figure 4, il n'est pas indispensable que le volume interne 4 ou les chambres telles que 44 aménagées dans celui-ci soient emplis d'un matériau offrant une flottabilité en cas d'avarie, ce rôle pouvant revenir au matériau 7 de remplissage de la chambre étanche 5 puisque, même en cas de déchirure de la paroi 2, ce matériau reste retenu, par exemple par le réseau de cloisons 9 s'il s'agit d'un matériau granulaire 8 comme on l'a illustré à la figure 2 ; ceci est vrai, par exemple, lorsqu'on utilise un matériau granulaire 8 sous forme de billes creuses, étanches aux gaz, d'un matériau expansé tel que du polystyrène expansé ; alors, il est possible d'assurer le remplissage du volume interne 4 constituant lui-même une chambre étanche ou des chambres étanches 44 aménagées dans ce volume interne exclusivement en air en légère surpression par rapport à la pression atmosphérique, ce qui autorise une fabrication puis une utilisation du flotteur ou du bâteau, ou autre dispositif glissant ou flottant conçu comme on l'a décrit en référence aux figures 3 et 4, de la façon suivante.

A la fabrication, après avoir assemblé les parois 2, 3, 28 ou 41 en prévoyant entre les parois 2, 3 le matériau de remplissage 7, et après avoir assemblé également à la paroi 2 les éventuelles parois telles que 31 et 49 destinées à délimiter avec elles des chambres étanches gonflables telles que 33 et 52, on commence par laisser la chambre interne 5 de la coque 1 ouverte à l'air libre par la valve 6 et, par la valve 35 ou 42 ou par les valves 45, on injecte dans le volume interne 4 ou dans les chambres 44, respectivement, le matériau fluide désiré tel que de l'air en légère surpression par rapport à l'air ambiant, si bien que la coque 1 prend sa conformation d'utilisation, telle qu'elle est illustrée à la figure 3 ou à la figure 4 par exemple ; on remarquera qu'il est possible d'intervenir sur cette conformation d'utilisation en provoquant alors un contact provisoire de la coque 1, vers l'extérieur, avec des moyens conformateurs appropriés tels que, par exemple, un gabarit d'installation dans le cas d'un réservoir ou d'un flotteur ou le corps d'un utilisateur en position d'utilisation dans le cas d'une coque de bateau ou d'une luge ; ensuite, par la valve 6, on place l'enceinte étanche 5 en dépression par rapport à l'air ambiant, ce qui plaque énergiquement les deux parois 2 et 3 sur le matériau de remplissage 7 et rigidifie ce dernier, c'est-à-dire la coque 1 dans son ensemble ; on peut ensuite gonfler à tout moment les chambres gonflables éventuellement prévues à l'extérieur de la coque 1, telles que la chambre 33 et la chambre 52.

Il est ensuite possible de réduire l'encombrement du dispositif lorsqu'il n'est pas utilisé, en rétablissant la pression atmosphérique à l'intérieur de l'enceinte 5 et en vidant le volume interne 4 ou les chambres 44, respectivement, ce qui permet de replier le flotteur, bâteau ou autre dispositif flottant ou glissant sous un volume réduit ; naturellement, il est nécessaire au préalable de dégonfler à nouveau les chambres telles que 33 et 52, extérieures à la coque 1.

Ensuite, en vue d'une nouvelle utilisation, on peut répéter les opérations initiales de mise en forme puis de rigidification de la coque 1 et de gonflage des chambres telles que 53 et 52.

Le réservoir illustré à la figure 1 peut être mis en forme de la même façon, par gonflage de la chambre 12 et, éventuellement des chambres 13 et 14 alors que l'enceinte 5 est ouverte à l'air libre puis mise en dépression relative de cette enceinte étanche 5 ; les chambres 12, 13, 14 peuvent ensuite être affectées à leur fonction respective de réception d'un fluide de commande et de réception d'un fluide à stocker.

On se réfèrera à présent à la figure 5, où l'on a illustré un dispositif selon l'invention sous forme d'un matelas anti-escarres de forme allongée, dont la coque 1 présente deux zones plates 54, 55, respectivement supérieure et inférieure, mutuellement parallèles, présentant des périphéries respectives 56, 57 le long desquelles elles sont raccordées mutuellement par une zone périphérique de flanc ou jupe 58 ; dans l'état du dispositif illustré à la figure 5, les zones 54 et 55 de même que la zone 58 présentent une symétrie par rapport à un plan longitudinal 71, vertical lorsque le dispositif est en service, mais il apparaîtra plus loin que cette symétrie n'est que temporaire.

De préférence, alors que les parois souples 2 et 3 de la coque 1 sont sensiblement inextensibles dans leurs zones correspondant à la zone inférieure 55 et à la zone périphérique de flanc 58, elles sont élastiquement extensibles dans leur zone correspondant à la zone supérieure 54, de façon à permettre au mieux le moulage de la coque 1 sur le corps d'un utilisateur comme il apparaîtra plus loin. De même, on peut choisir selon les zones des matériaux de remplissage 7 offrant des caractéristiques différentes, un tel choix pouvant d'ailleurs s'appliquer, selon les besoins, à tout mode de mise en oeuvre de la présente invention.

Comme la coque 1 des modes de mise en oeuvre de l'invention décrits en référence aux figures 3 et 4, la coque 1 de ce mode de mise en oeuvre de l'invention présente une discontinuité localisée dans sa zone inférieure 55, et plus précisément suivant le plan 71 le long duquel la coque 56 présente deux lèvres rectilignes 59, 60 qui sont toutefois disposées bord à bord, et raccordées par des moyens de liaison mutuelle tels qu'une fermeture à glissière ou des sangles, de façon non représentée, de telle sorte que la coque 1 enveloppe de toute part, sans discontinuité apparente, le volume interne 4 en assurant ou non une étanchéité de celui-ci. Naturellement, d'autres localisations pourraient être choisies pour cette discontinuité qui, en particulier, pourrait se situer dans la zone periphérique de flanc 58.

Avantageusement, la coque 1 est enveloppée vers l'extérieur par une housse souple 137 en contact intime avec sa paroi 2 ; cette housse est de préférence amovible et interchangeable, et peut par exemple présenter à cet effet en regard de la discontinuité précitée de la zone inférieure 55 une discontinuité analogue, fermée par des moyens tels qu'une fermeture à glissière ou des sangles, de façon non représentee mais aisément concevable par un Homme du métier ; la housse 137 peut ainsi assurer la fermeture de la coque 1 sur elle-même au niveau des lèvres 59 et 60, en remplacement des moyens précités de liaison mutuelle entre ces dernières ; naturellement, lorsque les parois souples 2 et 3 présentent un caractère élastiquement extensible dans leur zone correspondant à la zone supérieure 54 de la coque 1 et un caractère sensiblement inextensible par ailleurs, la housse 137 peut elle-même être élastiquement extensible en regard de la zone 54 de la coque 1 alors qu'elle peut être sensiblement inextensible par ailleurs. Une telle housse interchangeable permet d'améliorer l'hygiène d'utilisation du matelas ; elle permet aussi d'en améliorer le confort et peut présenter à cet effet, en regard de la zone 54 de la coque 1, tout revêtement approprié et/ou toute texture appropriée ; par exemple, de façon non représentée, elle peut présenter dans sa zone correspondant à la zone 54 de la coque 1 une structure multicouche connue en elle-même notamment dans le domaine des revêtements pour sièges de véhicules automobiles et comportant une couche souple de base en jersey élastiquement extensible, placée au contact de la coque 1 par la paroi 2 de celle-ci, une couche intermédiaire en matériau alvéolaire semi-rigide, élastiquement extensible et élastiquement compressible, ainsi que perméable à l'air, et une couche souple de revêtement en matériau textile élastiquement extensible et perméable à l'air, destinée au contact avec l'utilisateur, lesdites couches étant assemblées mutuellement par contrecollage thermique en compression ; naturellement, cet exemple ne doit pas être considéré comme limitatif.

Intérieurement, le volume interne 4, de forme générale aplatie, est intégralement occupé par trois chambres longitudinales étanches 61, 62, 63 délimitées par des parois souples, étanches respectives 64, 65, 66 qui peuvent être inextensibles ou élastiquement extensibles. La chambre 1, de forme générale aplatie et d'orientation générale perpendiculaire au plan 71 lorsque le dispositif se présente comme il est illustré à la figure 5, longe l'intégralité de la zone supérieure 54 de la coque 1 à l'intérieur du volume interne 4 ; intérieurement, elle est subdivisée par des cloisons souples, étanches 67, parallèles au plan 71 si l'on se réfère à la figure 5, de façon à délimiter en dessous de la totalité de la zone 54 un circuit 68 en serpentin, pour un fluide caloporteur tel qu'un liquide et par exemple de l'eau ou un gel, que des moyens 69 de circulation et de chauffage ou de réfrigération, extérieurs à la coque 1 et raccordés à la chambre 61 par des moyens appropriés formant valve, non représentés, font ainsi circuler à une pression qui peut être sensiblement identique ou supérieure à la pression atmosphérique, voire inférieure à celle-ci, pour, à volonté, réchauffer ou réfrigérer un patient reposant sur la zone supérieure 54 de la coque 1.

Naturellement, au lieu de s'étendre en continu sous l'intégralité de la zone supérieure 54 de la coque 1, la chambre 61 pourrait ne s'étendre que sur une zone limitée de celle-ci, en étant le cas échéant complétée par d'autres chambres analogues concernant d'autres parties de la zone supérieure 54 de la coque 1 et munie de leurs propres moyens 69 ; la chambre 61 ou chaque chambre analogue pourrait également être remplie d'un fluide statique tel qu'un liquide, ou un gaz ou mélange gazeux, ou un gel, ou un matériau particulaire, jouant exclusivement un rôle de répartition des pressions, auquel cas les moyens 69 seraient omis ; la chambre 61 et les moyens 69 pour y faire circuler un fluide caloporteur porté à une température désirée pourraient également être omis sans que l'on sorte pour autant du cadre de la présente invention ; en outre, la chambre 61 ou chaque chambre 61 pourrait être remplacée ou complétée par une chambre analogue, éventuellement associée à des moyens analogues aux moyens 69, et intégrée à la zone de la housse 137 placée en regard de la zone 54 de la coque 1, ou à une partie seulement de cette zone de la housse 137, de façon non illustrée à la figure 5 mais illustrée à la figure 6 en relation avec un autre mode de mise en oeuvre de l'invention qui sera décrit plus loin ; comme il ressortira de la description de cet autre mode de mise en oeuvre de l'invention, la ou chaque chambre analogue à la chambre 61 ou à chaque chambre analogue peut alors non seulement remplir les mêmes fonctions que la chambre 61 précédemment décrite ou chaque chambre analogue respectivement, mais également être utilisée à titre de coussin d'équi-répartition des pressions entre le corps de l'utilisateur et la coque 1 notamment après rigidification de celle-ci, pour en améliorer le confort, ou encore à titre de moyen de massage comme on le décrira ultérieurement en relation avec la figure 6.

Alors que la chambre 61 est symétrique par rapport au plan 71 dans l'état illustré du dispositif, les chambres 62 et 63 sont mutuellement symétriques par rapport à ce plan si l'on se réfère à la figure 5 et chacune d'entre elles présente une forme allongée de façon à s'étendre longitudinalement, d'un côté respectif du plan 71, entre une moitié respectivement correspondante de la chambre 61, une moitié respectivement correspondante de la zone périphérique de flanc ou jupe 58, et une moitié respectivement correspondante de la zone inférieure 55 de la coque 1, respectivement jusqu'à la lèvre 59 ou jusqu'à la lèvre 60 de celle-ci, lesquelles lèvres 59 et 60 sont disposées suivant le plan 71. Bien qu'elles puissent être emplies d'un matériau solide et élastiquement compressible tel qu'un matériau alvéolaire dans une version simplifiée du matelas illustré à la figure 5, les deux chambres 62 et 63 enferment un fluide qui peut être sensiblement incompressible, à savoir un liquide, ou sensiblement compressible élastiquement, à savoir un gaz ou mélange gazeux tel que de l'air, en surpression par rapport à la pression atmosphérique, et sont raccordées par des moyens formant valve, non illustrés, à des moyens de pompage ou moyens de gonflage et dégonflage 70 extérieurs à la coque 1 et permettant d'établir à volonté et de préférence de façon commandée automatiquement toute pression désirée dans chacune de ces chambres 62 et 63, notamment par transfert de liquide, gaz ou mélange gazeux de l'une à l'autre ; aux moyens de pompage 70 sont également associés des moyens raccordés à la valve 6 d'accès à l'enceinte étanche 5 pour permettre, à volonté et de préférence de façon commandée, de mettre cette enceinte étanche 5 à la pression atmosphérique en y laissant entrer un fluide tel qu'un gaz ou mélange gazeux ou un liquide, à la pression atmosphérique, ou de la ramener en dépression par rapport à la pression atmosphérique par extraction forcée de ce fluide, selon un cycle qui ressortira de la description, ci-après, de l'utilisation du matelas qui vient d'être décrit en référence à la figure 5.

Initialement, les deux chambres 62 et 63 de celui-ci sont remplies équivolumétriquement de liquide ou de gaz ou mélange gazeux porté à une même pression légèrement supérieure à la pression atmosphérique et l'éventuelle chambre 61 est emplie du fluide caloporteur ou du fluide statique qu'elle est destinée à recevoir ; par contre, l'enceinte 5 se trouve à la pression atmosphérique, si bien que la coque 1 présente de la souplesse.

Le matelas repose par sa zone 55 sur un support horizontal plan et on étend l'utilisateur sur sa zone 54, dans une position couchée considérée comme normale ; la zone 54 se déforme alors de telle sorte que, grâce à l'identité des pressions régnant dans les chambres 62 et 63 et par une transmission fidèle des pressions entre ces chambres et l'utilisateur par la zone 54 du matelas et par le fluide se trouvant dans la chambre 61, il y ait équirépartition des pressions de contact entre l'utilisateur et la paroi 2 de la coque 1 qui est ensuite rigidifiée par mise en dépression relative.

Au bout d'un temps prédéterminé par une minuterie incorporée aux moyens 70 ou sous l'action d'autres moyens de commande automatique intégrés aux moyens 70 et répondant par exemple à des signaux provenant de capteurs de pression de contact répartis sur la zone 54 de la coque 1 ou sur la zone correspondante de la coque 137 de façon à établir en permanence un spectre de répartition des pressions de contact entre le corps de l'utilisateur et le matelas pour commander, en fonction d'un programme enregistré, des modifications de la conformation de la zone 54 de la coque 1 propres à corriger ce spectre de répartition des pressions de contact, de façon prédéterminée en fonction de données de valeur de pression de contact, de durée et de surface d'application de pression de contact, ainsi que d'un historique de ces données, de façon aisément concevable par un Homme du métier, ou encore à la demande de l'utilisateur constatant une douleur localisée, et grâce aux moyens 70, on peut ensuite rétablir la pression atmosphérique dans l'enceinte étanche 5 puis provoquer une modification des volumes apparents respectifs des chambres 62 et 63 par exemple par un transfert de liquide, gaz ou mélange gazeux de l'une à l'autre des chambres 62 et 63, ce qui provoque le basculement de la zone 54 de la coque 1 et, avec elle, de l'utilisateur couché sur cette zone 54, puis on rétablit une dépression relative dans l'enceinte étanche 5, pour rigidifier la zone 54 dans la nouvelle conformation. Ensuite, après un temps déterminé par une minuterie incorporée aux moyens 70, ou sous l'action desdits autres moyens de commande automatique, ou à la demande de l'utilisateur constatant à nouveau une douleur, on peut à nouveau par les moyens 70 mettre l'enceinte 5 à la pression atmosphérique pour communiquer une souplesse à la coque 1, puis provoquer une nouvelle modification des volumes apparents respectifs des chambres 62 et 63 par exemple par un nouveau transfert de liquide, de gaz ou de mélange gazeux de l'une à l'autre des chambres 62 et 63, toutefois en sens opposé au sens du transfert précédent, ce qui modifie la position de l'utilisateur en même temps que celle de la zone 54, puis on rétablit la dépression relative dans l'enceinte étanche 5 pour rigidifier à nouveau la coque 1 dans sa nouvelle conformation.

Un tel cycle peut être répété en permanence, de préférence de façon automatisée, ce qui évite à l'utilisateur de reposer toujours par les mêmes zones du corps sur la zone supérieure 54 du matelas, et permet d'éviter ainsi les escarres ; une circulation de fluide dans la chambre 61 contribue au confort d'utilisation du matelas.

On se réfèrera à présent à la figure 6 où l'on a illustré un matelas selon l'invention, destiné à un usage chirurgical, c'est-à-dire à soutenir et caler le corps d'un patient pendant une intervention chirurgicale, ainsi qu'avant et après cette intervention.

La coque 1 de ce matelas présente une forme analogue à celle du matelas illustré à la figure 5 et comporte notamment des zones supérieure 72, inférieure 73 et périphérique 74 en tout point analogues aux zones 54, 55, 58 respectivement, si ce n'est qu'au niveau de la zone 73, la coque 1 présente une discontinuité localisée entre des lèvres longitudinales 75, 76 mutuellement écartées, symétriques l'une de l'autre par rapport à un plan longitudinal 77 constituant un plan longitudinal de symétrie pour chacune des zones 72 et 73 ainsi que pour la zone 74, et entre des lèvres transversales non visibles à la figure 5, également mutuellement écartées et quant à elles respectivement symétriques par rapport au plan 77 ; ce plan 77 est vertical lorsque le matelas est en cours d'utilisation, les zones 72 et 73 étant quant à elles horizontales de même que les lèvres 75 et 76.

Toutefois, les lèvres longitudinales 75 et 76 sont reliées mutuellement par une pluralité de liens souples 78 transversaux, de préférence élastiquement extensibles, de même que les lèvres transversales non représentées sont reliées mutuellement par une pluralité de liens souples 79 longitudinaux, de préférence élastiquement extensibles.

Le volume interne 4 de la coque rigide 1 est occupé par une juxtaposition transversale, à plat, de chambres étanches 81, 82 délimitées par des parois souples, étanches respectives 83, 84 inextensibles ou élastiquement extensibles ; ces chambres 81 et 82 sont associées, par l'intermédiaire de valves d'accès non représentées, à des moyens 85 analogues aux moyens 70 en ce qu'ils permettent d'établir toute pression désirée dans l'une ou l'autre de ces chambres, et de placer l'enceinte étanche 5 alternativement à la pression atmosphérique ou en dépression par rapport à la pression atmosphérique.

Toutefois, de façon localisée à l'intérieur du volume interne 4, sur certains des liens souples 78, 79 repose de préférence librement une protubérance transversale 86 qui peut être rigide, sensiblement incompressible, et par exemple constituée d'un bloc rigide approximativement parallélépipédique, de façon non illustrée, ou de préférence élastiquement compressible, et par exemple constituée par une enveloppe souple sensiblement inextensible 149 délimitant une chambre étanche 150 analogue aux chambres 81 et 82 et gonflée en surpression sensible par rapport à la pression atmosphérique, comme on l'a illustré, sur laquelle la zone supérieure 72 de la coque 1 s'appuie localement si bien qu'elle bénéficie ainsi d'un appui sur tout support généralement plan et horizontal 181, tel qu'une table d'opération, sur lequel la zone inférieure 73 de la coque 1 et les liens 78, 79 reposent vers le bas ; la protubérance 86 est par ailleurs enveloppée de toute part par les chambres 81 et 82, par rapport auxquelles elle constitue pour la zone 72 un appui localisé, de fermeté prédéterminée, en un emplacement déterminé par l'emplacement du lien souple 78 auquel on l'associe, pour constituer un billot.

On conçoit aisément qu'en réglant de façon déterminée les pressions dans les chambres 81 et 82 entourant le billot constitué par la protubérance localisée 86 ainsi que dans cette dernière lorsque sa nature s'y prête, alors que l'enceinte 5 se trouve à la pression atmosphérique, on peut donner à la zone supérieure 72 de la coque 1 toute conformation propre à placer le patient dans la posture la mieux appropriée puis, ensuite, figer la coque 1 dans la position obtenue par mise en dépression relative de l'enceinte étanche 5.

Un tel matelas présente un avantage important, par rapport à d'autres types de matelas utilisés en chirurgie, en ce que la rigidité qu'il présente lorsque la coque 1 se trouve à l'état rigide, c'est-à-dire lorsque l'enceinte étanche 5 se trouve en dépression relative, permet d'y placer le patient dans la position requise avant d'avoir placé le matelas sur la table d'opération, puis d'amener le matelas avec le patient sur la table d'opération sans avoir à déplacer le patient par rapport au matelas, de pratiquer ainsi l'opération puis, après l'opération, d'amener le patient en salle de réanimation toujours sans le déplacer par rapport au matelas ; à cet effet, on prévoit avantageusement de part et d'autre du matelas des poignées de portage telles que 80, par exemple constituées par des extrémités de sangles transversales, souples et sensiblement inextensibles, engagées sous la zone inférieure 73 de la coque 1 et sous les liens souples 78, 79, par exemple par l'intermédiaire de passants appropriés solidaires de la paroi 2 de l'enceinte étanche 4 et non illustrés.

Naturellement, bien que cela n'ait pas été représenté, le mate!as illustré à la figure 6 pourrait présenter, comme celui qui a été illustré à la figure 5, des moyens analogues à la chambre 61 et permettant de retenir un fluide contre sa zone 72 ou de faire circuler un fluide caloporteur contre sa zone 72 avant et/ou pendant et/ou après l'opération, soit à l'intérieur du volume 4, notamment grâce à la présence d'au moins une chambre analogue à la chambre 61 et interposée entre la zone supérieure 72 de la coque 1, d'une part, et la protubérance 86 ainsi que les chambres 81 et 82, d'autre part, soit à l'extérieur du volume 4, et plus précisément en regard de la zone de la paroi 2 correspondant à la zone 72 de la coque 1, grâce à la présence d'au moins une chambre analogue à la chambre 61 et par exemple intégrée à une housse souple 138 enveloppant extérieurement la coque 1 en épousant étroitement la paroi 2 de celle-ci.

La housse 138, de préférence sensiblement inextensible, présente une grande analogie avec la housse 137 décrite en référence à la figure 5 en ce qu'elle enveloppe étroitement, par la paroi 2 de l'enceinte 1, la coque rigide 1 dans sa zone supérieure 72, sa zone périphérique 74, et sa zone inférieure 73, en regard de la discontinuité de laquelle elle présente toutefois elle-même une discontinuité localisée entre deux lèvres longitudinales 139, 140 mutuellement écartées, symétriques l'une de l'autre par rapport au plan 77 et raccordées mutuellement par des liens souples transversaux tels que 141 élastiquement extensibles, et deux lèvres transversales non visibles à la figure 6, mutuellement écartées, quant à elles respectivement symétriques par rapport au plan 77 et raccordées mutuellement par des liens souples longitudinaux tels que 142 élastiquement extensibles ; on remarquera que les sangles constituant les poignées de portage 80 peuvent être portées par la housse 138, par exemple par l'intermédiaire de passants non représentés, plutôt que par la coque 1 elle-même.

En regard de l'intégralité ou d'une partie prédéterminée de la zone supérieure 72 de la coque 1, la housse 138 délimite par deux parois souples, étanches 182 et 183 une chambre étanche 143 qui, comme la chambre 61, présente une forme aplatie et une orientation générale sensiblement perpendiculaire au plan 77 dans la conformation illustrée du matelas et pourrait être cloisonnée comme la chambre 61 pour recevoir un fluide statique de répartition de pression ou un fluide caloporteur circulant.

Toutefois, dans l'exemple illustré, la chambre 143 est cloisonnée de façon étanche, par des cloisons souples transversales 144, en une multitude de chambres étanches transversales 145, mutuellement indépendantes, qui sont emplies d'un fluide tel qu'un liquide ou un gaz ou mélange gazeux et dont chacune présente des moyens propres d'accès sous la forme d'une valve respective 146 raccordée par une conduite respective 147 à un automate 148 permettant d'établir selon une séquence prédéterminée des pressions respectives prédéterminées, variables dans le temps de façon prédéterminée entre des valeurs maximales qui peuvent être sensiblement supérieures à la pression atmosphérique et des valeurs minimales qui peuvent être de l'ordre de la pression atmosphérique, dans le fluide emplissant respectivement chaque chambre 145, de façon à induire un effet de massage du corps de l'utilisateur ; naturellement, d'autres moyens pourraient être utilisés en vue d'une telle action sur les pressions régnant dans les chambres 145 respectivement ; le massage obtenu est d'autant plus efficace que, dès lors que la coque 1 est mise en forme directement sur le corps de l'utilisateur de la façon précédemment décrite, par l'intermédiaire de la chambre 143 placée en équipression lors de cette mise en forme, il s'établit entre le corps de l'utilisateur et le matelas un contact continu tel que les variations de pression dans les chambres 145 se transmettent fidèlement aux zones respectivement correspondantes du corps.

Naturellement, au lieu d'être rapportée sur la coque 1 par l'intermédiaire de la housse 138, la chambre subdivisée 143 pourrait être aménagée directement sur la coque 1, par exemple entre la paroi 2 et une paroi souple, étanche, doublant celle-ci vers l'extérieur du matelas au niveau de la zone supérieure 72 de la coque 1.

Comme on l'a dit précédemment, une chambre subdivisée analogue à la chambre 143 et associée comme celle-ci à un automate tel que l'automate 148, permettant d'induire un effet de massage, pourrait être prévue également dans le cas du matelas illustré à la figure 5, soit directement sur la coque 1 de celui-ci, soit au niveau de sa housse 137.

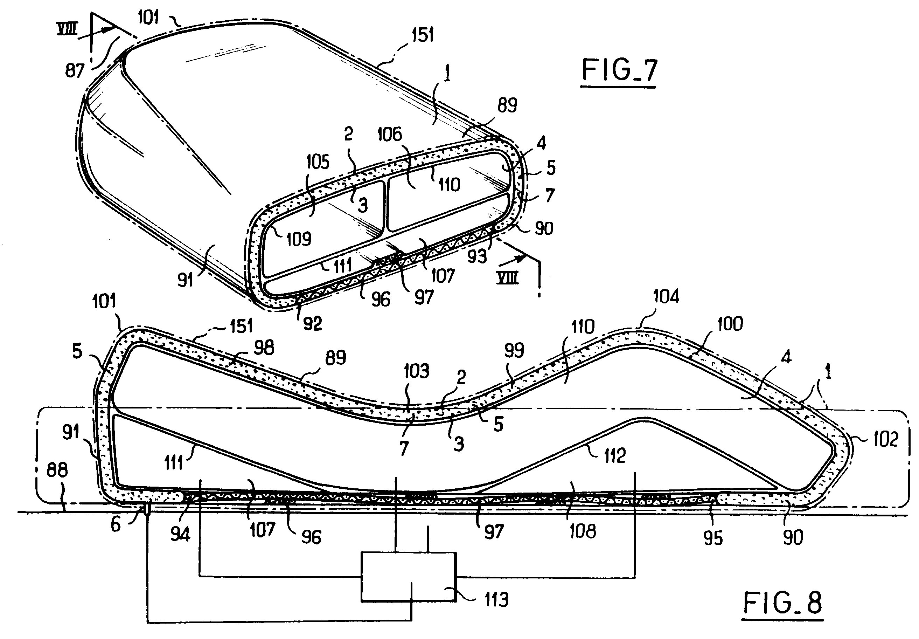

Une telle chambre, ou encore une chambre permettant de recevoir un fluide statique de répartition de pression ou un fluide caloporteur circulant comme on l'a décrit en référence à la figure 5 soit à l'intérieur du volume interne 4 de la coque 1, soit vers l'extérieur de celle-ci, pourrait également être avantageusement prévue, bien que celà n'ait pas été représenté, dans un matelas anti-escarres qui va être décrit à présent en référence aux figures 7 et 8, et qui constitue par ailleurs une version plus complète du matelas décrit en référence à la figure 5.

Tel qu'il se présente aux figures 7 et 8, ce matelas anti-escarres présente une symétrie générale par rapport à un plan longitudinal 87, vertical lorsque ce matelas repose à plat sur un support plan, horizontal 88, de préférence rigide.

La coque 1 de ce matelas présente une grande analogie avec la coque 1 du matelas décrit en référence à la figure 6, en ce qu'elle présente une zone supérieure 89 et une zone inférieure 90 raccordées mutuellement par une zone périphérique 91 et en ce que, alors que les zones 89 et 91 sont continues, la zone 90 présente une discontinuité délimitée par deux lèvres longitudinales 92, 93 mutuellement espacées et mutuellement symétriques par rapport au plan 87, et par deux lèvres transversales 94, 95 mutuellement espacées et dont chacune est symétrique par rapport au plan 87 ; les deux lèvres 92 et 93 sont raccordées mutuellement par une pluralité de liens souples 96 transversaux, élastiquement extensibles entre les lèvres 92 et 93, et les lèvres transversales 94 et 95 sont raccordées mutuellement par une pluralité de liens souples longitudinaux 97, élastiquement extensibles entre elles, ces liens souples 96 et 97 étant choisis tels qu'ils restent tendus quelles que soient les conformations que l'on peut donner à la coque 1 dans les limites d'utilisation du matelas qui seront décrites ultérieurement.

La coque 1 est de préférence enveloppée d'une housse souple 151 non détaillée mais en tout point analogue à la housse souple 138 notamment quant à son mode d'enveloppement des zones 89, 90, 91 du matelas, quant à sa discontinuité inférieure, entre des lèvres reliées mutuellement par des liens souples élastiquement extensibles non représentés, et quant au fait qu'elle peut se présenter sous les différents modes de réalisation décrits en référence aux figures 5 et 6, à propos des housses 137 et 138 respectivement, et notamment délimiter en regard de la zone supérieure 89 de la coque 1 une chambre étanche apte à recevoir un fluide statique de répartition de pression ou un fluide caloporteur circulant, ou un fluide permettant d'induire un effet de massage.

Alors que la zone inférieure 90 du matelas est plate, et repose en pratique à plat sur le support 88, le cas échéant par l'intermédiaire de la housse 138 et de liens souples, élastiquement extensibles fermant une discontinuité de celle-ci, sa zone supérieure 89 comporte trois parties 98, 99, 100 dont chacune est plate, perpendiculaire par rapport au plan 87 et symétrique par rapport à celui-ci dans deux conformations illustrées, et qui se succédent longitudinalement d'une zone d'extrémité transversale 101 du matelas à une autre zone 102 d'extrémité transversale de celui-ci ; ces trois parties 98, 99, 100 peuvent être disposées selon un même plan moyen parallèle au support 88, pour communiquer à la zone supérieure 89 de la coque 1 une forme générale plate, dans une conformation du matelas illustrée en trait mixte à la figure 8 ; cependant, grâce à des moyens qui seront décrits plus loin, on peut également donner au matelas une conformation dans laquelle la zone supérieure 89 de la coque 1 présente une forme ondulée et qui a été illustrée en trait plein aux figures 7 et 8 ; cette dernière conformation servira de référence à la suite de la description.

Longitudinalement, la partie 98 de la zone supérieure 89 de la coque 1 présente des dimensions telles qu'elle puisse servir de support à un corps humain depuis la tête, jouxtant la zone d'extrémité 101, jusqu'au bassin, situé dans une partie 103 de jonction entre les parties 98 et 99 ; cette partie 98 descend progressivement de la zone d'extrémité 101 à la zone de jonction 103 ; longitudinalement, la partie 99, ascendante de la partie 103 de jonction avec la partie 98 à une partie 104 de jonction avec la partie 100, présente des dimensions correspondant sensiblement à celles qui séparent le bassin du genou, pour recevoir les cuisses de l'utilisateur, alors que la partie 100, descendante de la partie 104 de jonction avec la partie 99 jusqu'à la zone d'extrémité 102 du matelas, présente des dimensions correspondant aux dimensions séparant le genou du pied pour servir de support aux mollets et aux pieds de l'utilisateur ; la partie de jonction 103, concave, et la partie de jonction 104, convexe, présentent des formes arrondies.

Comme il apparaîtra plus loin, la géométrie des différentes parties 98, 99, 100, 103, 104 de la coque 1 peut être modifiée en fonction des besoins.

Dans le cas de ce matelas, le volume interne 4 de la coque 1 est occupé par quatre chambres étanches 105, 106, 107, 108 dont chacune est délimitée par une paroi respective 109, 110, 111, 112 souple, étanche, de préférence élastiquement extensible, et raccordée par des valves respectives (non représentées) à des moyens 113 de gonflage et de dégonflage commandés, auxquels est également raccordée la valve 6 d'accès à l'enceinte étanche 5 de la coque 1 en vue d'un fonctionnement qui sera décrit plus loin.

Les deux chambres étanches 105 et 106 présentent la forme de boudins longitudinaux mutuellement juxtaposés transversalement, à plat, immédiatement en dessous de la zone supérieure 89 de la coque 1 et, dans la conformation illustrée à la figure 7, sont mutuellement symétriques par rapport au plan 87 ; chacun de ces boudins s'étend ainsi transversalement du plan 87 jusqu'à la zone périphérique 91 de la coque 1 et longitudinalement de la zone d'extrémité 101 du matelas jusqu'à la zone d'extrémité 102 de celui-ci, en jouxtant dans ces deux zones la zone périphérique 91 de la coque 1 comme il ressort de l'examen de la figure 8, montrant la paroi 110 étant entendu que la paroi 109 en est symétrique par rapport au plan 87 ; les deux boudins longitudinaux ainsi constitués sont mutuellement jointifs, par les parois 109 et 110, le long du plan 87.

Entre les deux chambres 105 et 106 et un ensemble constitué par la zone inférieure 90 de la coque 1 et par les sangles 96 et 97 sont interposées localement les deux autres chambres 107 et 108, qui présentent quant à elles la forme de boudins transversaux dont le premier est localisé à l'aplomb de la partie 98 de la zone supérieure 89 alors que le second est localisé à l'aplomb des parties 99, 100 et 104 de cette zone supérieure 89 de façon à communiquer aux parties 98, 99, 100 de la zone supérieure 89 de la coque 1 la conformation indiquée ci-dessus ; transversalement, les deux boudins transversaux ainsi constitués s'étendent symétriquement par rapport au plan 87 pour jouxter de part et d'autre de celui-ci la zone périphérique 91 de la coque 1 ; lorsqu'ils sont vus en coupe par un plan longitudinal, comme c'est le cas de la figure 8, ils présentent une section sensiblement triangulaire, à savoir respectivement une section en triangle rectangle dont l'hypothénuse jouxte les parois 109, 110 des chambres 105 et 110 alors que les côtés de l'angle droit jouxtent respectivement la zone périphérique 91 de la coque 1 et l'ensemble formé par la zone inférieure 90 de celle-ci et par les sangles 96, 97, et une section en triangle isocèle dont la base jouxte cet ensemble et dont les deux autres côtés jouxtent les parois 109 et 110, le sommet étant disposé à l'aplomb de la partie 104 de jonction entre les parties 99 et 100 de la zone supérieure 89 de la coque 1 ; dans l'exemple illustré, les deux chambres 107 et 108 sont mutuellement disjointes à l'aplomb de la partie 103 de jonction entre les parties 98 et 99 de la zone supérieure 89 de la coque 1 du matelas, si bien que les parois 105 et 106 reposent directement sur l'ensemble formé par la zone inférieure 90 de la coque 1 et par les sangles 96 et 97 mais on pourrait également prévoir que, tout en restant mutuellement indépendantes, les chambres 107 et 108 soient mutuellement jointives à l'aplomb de cette partie 103, ou encore prévoir entre les chambres 107 et 108 une chambre transversale supplémentaire, sous forme d'un boudin transversal localisé à l'aplomb de la partie 103, sous celle-ci, et en tout point analogue aux chambres 107 et 108 dont cette chambre supplémentaire serait indépendante notamment quant à son gonflage et quant à son dégonflage par les moyens 113.

Naturellement, on ne sortirait pas du cadre de la présente invention en inversant les positions respectives des boudins longitudinaux et des boudins transversaux, c'est-à-dire en interposant ces derniers entre les boudins longitudinaux et la zone supérieure 89 de la coque 1.

Le fonctionnement du matelas illustré aux figures 7 et 8 peut être le suivant, étant entendu que ce fonctionnement peut être commandé manuellement ou automatiquement par les moyens 113, qui peuvent être munis à cet effet d'une minuterie ou d'autres moyens de commande répondant par exemple à des signaux provenant de capteurs de pression de contact répartis sur la zone supérieure 89 de la coque 1 pour établir en permanence un spectre de répartition des pressions de contact entre le corps de l'utilisateur et cette zone 89 et commander, en fonction d'un programme enregistré, des modifications de la conformation de la zone supérieure 89 de la coque 1 propres à corriger ce spectre de pressions de contact, de façon déterminée en fonction de données de valeur de pression de contact, de durée et de surface d'application de pression de contact, ainsi que d'un historique de ces données.

Dans une première phase d'utilisation du matelas, correspondant à sa mise en service, la chambre étanche de la coque 1 est mise à la pression ambiante, ce qui communique à la coque 1 une souplesse, et les chambres 105, 106, 107, 108 sont placées en équi-pression, puis on étend l'utilisateur sur la zone supérieure 89 du matelas ; cette zone supérieure 89 se conforme alors selon le corps de l'utilisateur, de façon à assurer une équi-répartition des pressions, qui se transmettent fidèlement entre les chambres 105 et 106, d'une part, et le corps de l'utilisateur, d'autre part ; en gonflant plus ou moins, de façon éventuellement différente, les chambres 107 et 108, on peut orienter l'une par rapport à l'autre les différentes parties 98, 99, 100 de la zone supérieure 89 du matelas en conservant cette équi-répartition des pressions ; on peut ainsi notamment dégonfler totalement les chambres 107 et 108 pour donner au matelas la conformation illustrée en trait mixte à la figure 8 ; lorsque le matelas présente la conformation désirée, on met l'enceinte étanche 5 en dépression par rapport à la pression atmosphérique, ce qui rigidifie la coque 1 dans la conformation obtenue ; on remarquera que lorsque la coque 1 se trouve à l'état souple, les sangles 96 et 97 la maintiennent en tension autour des chambres étanches 105, 106, 107, 108.

Ensuite, de façon automatique ou commandée manuellement, par exemple dans le cas de douleurs localisées de l'utilisateur, on peut à nouveau mettre l'enceinte 5 à la pression atmosphérique puis modifier la conformation de la zone supérieure 89 de la coque 1 par exemple en plaçant l'une des chambres 105, 106 en surpression par rapport à l'autre, ce qui fait disparaître la symétrie de la zone 89 par rapport au plan 87 en la faisant basculer par rapport à celui-ci, ou en modifiant les gonflages respectifs des chambres 107 et 108 ; lorsqu'une nouvelle conformation désirée est obtenue, on rigidifie à nouveau la coque 1 en mettant l'enceinte étanche 5 en dépression relative ; l'opération inverse peut être ensuite effectuée, comme on l'a expliqué en référence à la figure 5.

Le recours à des moyens automatiques pour commander le fonctionnement du matelas permet de gérer la répartition statistique, dans le temps, des zones d'appui du corps de l'utilisateur sur le matelas et des pressions de contact mutuel dans ces zones, en faisant travailler non seulement les zones d'appui naturel du corps mais également des zones qui ne servent normalement pas d'appui, telles que le dessous des genoux, pour s'assurer de ce que dans chaque zone du corps, la pression de contact mutuel et la durée d'application de cette pression soient en permanence propres à éviter non seulement la douleur à court terme, mais également l'apparition d'escarres à plus long terme, ou encore le traitement d'escarres pré-existantes.

Naturellement, bien que, dans les différents modes de réalisation d'un matelas selon l'invention qui ont été décrits en référence aux figures 5 à 8, on ait décrit un remplissage intégral des chambres étanches intérieures au volume interne 4 de la coque 1 en matériau fluide, on pourrait également prévoir que ces dernières contiennent en outre un matériau expansé, élastiquement compressible, en bloc ou sous forme particulaire, les emplissant partiellement ou totalement pour contribuer à la conformation de la coque 1 lorsque celle-ci se trouve à l'état souple, communiquer au matelas une forme de base dans une telle hypothèse et lorsqu'en outre les chambres étanches intérieures sont placées à la pression atmosphérique, et limiter les quantités de matériau fluide à déplacer pour, respectivement, emplir ou vider ces chambres ; on pourrait également utiliser à ces différents effets des matériaux ou dispositifs placés directement à l'intérieur du volume interne 4, et notamment des dispositifs du type décrit dans WO 87/06209 précité, offrant en eux-mêmes une possibilité de conformation à volonté ; en outre, bien que, en référence aux figures 5 à 8, on ait décrit seulement deux chambres longitudinales, on pourrait prévoir de telles chambres en un nombre supérieur, à l'état juxtaposé transversalement.

On pourrait également prévoir les chambres 62, 63, 81, 82, 105, 106, 107, 108 décrites en référence aux figures 5 à 8 en un nombre différent et sous une forme différente, et par exemple sous la forme d'une multitude de plots mutuellement juxtaposés longitudinalement comme transversalement, sous la zone supérieure 54, 72, 89 de la coque 1, pour autoriser une adaptation plus fine de la conformation de la coque 1 aux besoins, lorsque l'enceinte 5 est à la pression atmosphérique, en coopérant à cet effet avec des moyens 70, 148, 113 convenablement adaptés au nombre de ces chambres et propres à communiquer à chacune d'entre elles tout volume respectif voulu, c'est-à-dire toute dimension voulue perpendiculairement à la zone supérieure 54, 72, 89 de la coque 1, par gonflage en surpression de préférence faible par rapport à la pression atmosphérique ou dégonflage, par exemple en fonction de données mémorisées.

Le cas échéant, lorsque le volume interne 4 est fermé de façon étanche soit par raccordement mutuel étanche des lèvres 59 et 60 de la coque 1 dans le cas du mode de mise en oeuvre illustré à la figure 5, soit par tout moyen approprié tel qu'un voile étanche, élastiquement extensible, se raccordant de façon étanche et de préférence amovible aux lèvres longitudinales 75, 76 et aux lèvres transversa!es non représentées dans le cas du mode de mise en oeuvre illustré à la figure 6 ou aux lèvres longitudinales 92, 93 et aux lèvres transversales 94, 95 dans le cas du mode de mise en oeuvre illustré aux figures 7 et 8, de façon non représentée mais aisément concevable par un Homme du métier, on peut également prévoir d'adapter étroitement la conformation de la coque 1, lorsque l'enceinte 5 se trouve à la pression atmosphérique, par tirage au vide à l'intérieur du volume interne 4, entre la paroi 3 délimitant l'enceinte 5 vers celui-ci, d'une part, et les parois respectives 64, 65, 66, 83, 84, 109, 110, 111, 112 des chambres 61, 62, 63, 81, 82, 105, 106, 107, 108 de même que la protubérance 86, d'autre part, grace aux moyens 70, 148, 113 respectivement, alors convenablement raccordés à cet effet au volume interne 4, de façon non représentée mais aisément concevable par un Homme du métier, et convenablement commandés à cet effet. Aux cycles de fonctionnement précités s'ajouteraient alors une phase de mise en dépression du volume interne 4 entre le changement de conformation des chambres 62, 63, 81, 82, 105, 106, 107, 108 et la mise en dépression de l'enceinte 5, et une phase d'établissement de la pression atmosphérique dans le volume interne 4 à tout moment voulu dès lors qu'il serait préalable à un nouveau changement de conformation desdites chambres.

De même, dans une version simplifiée d'un matelas selon l'invention, on pourrait prévoir à l'intérieur de la coque 1 une chambre étanche, gonflable, unique comme on l'a décrit par exemple à propos du flotteur illustré à la figure 3 ou du bâteau illustré à la figure 4.

Toutefois, dans le cas d'une application de l'invention au calage et/ou soutien du corps, on préfère limiter le recours à une telle chambre intérieure unique au cas de dispositifs de dimensions inférieures à celles d'un mate!as, c'est-à-dire au cas de coussins.

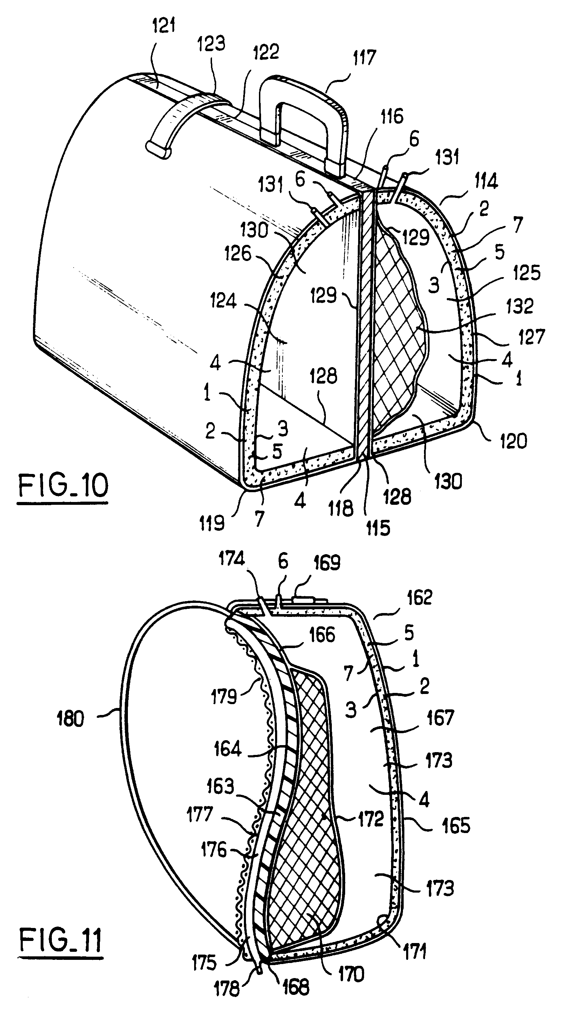

On a précisément illustré à la figure 9 un coussin d'assise qui, si l'on se réfère à sa position d'utilisation, est aplati en direction verticale et symétrique par rapport à un plan vertical 152 et dont la conception s'apparente à celle du flotteur illustré à la figure 3 en ce que la coque rigide 1 enveloppe le volume interne 4 de façon continue de toute part, sauf vers le bas, c'est-à-dire dans une zone inférieure 153 du coussin dans laquelle la coque rigide 1 se raccorde de façon continue et étanche, par une lèvre annulaire 154, à une paroi 155 étanche, rigide ou de préférence élastiquement déformable en compression, en flexion et en torsion, qui coopère ainsi avec la coque 1 pour enfermer de toute part, de façon étanche, le volume interne 4. Ce volume interne 4 constitue ainsi lui-même une chambre étanche accessible par une valve 156 permettant d'y introduire ou d'en extraire un fluide, par exemple un liquide ou un gaz ou mélange gazeux tel que de l'air à une pression proche de la pression ambiante, c'est-a-dire de la pression atmosphérique, et de préférence en légère surpression, ou encore un gel.

Dans des variantes de réalisation du coussin illustré à la figure 9, on pourrait cependant prévoir que le volume interne 4 de la coque rigide 1 soit compartimenté de façon étanche, par des parois y délimitant des chambres étanches, munies de valves respectives d'accès, à la façon dont les parois 15, 16, 17 délimitent des chambres étanches 12, 13, 14 dans le cas du réservoir illustré à la figure 1.

La paroi 155, par laquelle le coussin repose sur tout support approprié tel qu'une chaise ou un fauteuil, présente vers le bas, c'est-à-dire à l'opposé du volume interne 4, toute forme appropriée à cet effet et par exemple une forme générale plate bordée d'un rebord convexe se raccordant à la coque 1 au niveau de la lèvre 154.

Vers le haut, c'est-à-dire vers l'intérieur du volume interne 4, la paroi 155 présente une forme anatomique, à savoir un relief caractérisé par une protubérance 158 localisée le long du plan 152 entre deux zones en creux relatif 159, 160 disposées symétriquement l'une de l'autre en référence au plan 152, de telle sorte que même si la chambre constituée par le volume 4 est vide et la coque 1 à l'état souple par mise de l'enceinte 5 à la pression atmosphérique, l'utilisateur reposant sur la paroi 155 par l'intermédiaire de la coque 1 à l'état souple soit néanmoins placé dans des conditions convenables, sinon optimales, de confort et de maintien par le coussin ; en outre, la présence de la protubérance 158 à l'intérieur du volume interne 4 permet de réduire le volume de matériau fluide à déplacer pour remplir ou vider celui-ci et en particulier, lorsque ce matériau est un gaz ou mélange gazeux ou encore un liquide également utilisé pour mettre l'enceinte 5 à la pression atmosphérique par introduction de ce matériau dans celle-ci ou pour tirer au vide dans l'enceinte 5 par extraction de ce matériau hors de celle-ci, d'équilibrer sensiblement les volumes de matériau fluide qu'il faut respectivement introduire dans le volume 4 pour gonfler celui-ci et extraire de l'enceinte 5 pour rigidifier la coque 1, ou extraire du volume 4 pour dégonfler celui-ci et introduire dans l'enceinte 5 pour assouplir la coque 1, par l'intermédiaire de moyens 161 de pompage et de refoulement, ou de gonflage et de dégonflage auxquels sont raccordées les valves 6 et 156.

Un tel coussin peut être utilisé de la façon suivante :
- alors que la coque 1 est à l'état souple et que la chambre étanche définie par le volume interne 4 est gonflée du matériau fluide choisi, de préférence un gaz ou mélange gazeux ou un liquide, le coussin reposant par la paroi 155 sur le support qui est destiné à le recevoir, l'utilisateur s'assoit en position normale sur la coque 1 qui se moule sur lui, le matériau emplissant la chambre définie par le volume 40 assurant une équi-répartition des pressions d'appui du corps de l'utilisateur sur la coque 1 ;
- ensuite, on rigidifie la coque 1 dans la conformation obtenue, et on peut alors soit maintenir dans le volume interne 4 le matériau ayant servi précédemment à gonfler ce volume, en modifiant éventuellement sa pression par augmentation ou réduction, soit extraire ce matériau du volume interne 4, en lui substituant éventuellement un autre matériau fluide, éventuellement susceptible de faire ensuite prise en se rigidifiant ou en conservant une compressibilité élastique et/ou une souplesse, comme par exemple une résine synthétique expansible.

Extérieurement, le coussin est avantageusement enveloppé, au moins en regard de la coque 1 qui en constitue la partie destinée à venir au contact du corps de l'utilisateur, d'une housse amovible 157 en améliorant le confort de contact et d'utilisation en longue durée, et pouvant présenter à cet effet les différentes caractéristiques des housses 137, 138, 151 décrites en référence aux figures 5 à 8.

Naturellement, le mode de réalisation d'un coussin illustré à la figure 9 ne constitue qu'un exemple non limitatif de coussin susceptible de mettre en oeuvre la présente invention et, en particulier, on pourrait prévoir d'en subdiviser le volume interne 4 en plusieurs chambres étanches mutuellement indépendantes éventuellement associées à un automate ou à des moyens commandés à volonté pour gonfler et de dégonfler celles-ci, comme on l'a décrit en référence aux figures 5 à 8 à propos de matelas.

On se réfèrera à présent à la figure 10, où l'on a décrit l'application de l'invention à la réalisation d'un dispositif de calage et de soutien d'objets dans un sac de voyage, à titre d'exemple non limitatif de moyens définissant une cavité interne dans laquelle on peut souhaiter caler un ou plusieurs objets.

Le sac de voyage 114 illustré à la figure 10 présente. si l'on se réfère à la position illustrée, une paroi médiane 115 plate, verticale, rigide, présentant notamment un bord supérieur 116 horizontal, muni d'une poignée de préhension 117, et un bord inférieur également horizontal 118 ; le long de ce bord inférieur 118 et de deux bords verticaux, non représentés, de la paroi 115 sont fixées sur celle-ci, respectivement de part et d'autre de celle-ci, deux parois latérales 119, 120 quant à elles souples et de forme enveloppante, lesquelles parois 119, 120 présentent un bord supérieur respectif 121, 122, libre, susceptible d'être soit accolé à la paroi médiane 115 le long du bord supérieur 116 de celle-ci, sous l'action de moyens de fermeture 123 tels que des sangles munies de serrures, pour fermer le sac, ou au contraire être écartées du bord supérieur 116 de la paroi 115 lorsqu'on désire ouvrir le sac ; la conformation et la réalisation pratique du sac 114 ne sont pas critiques quant à la présente invention et peuvent être choisies dans une large gamme de possibilités, si bien que la sac 114 ne sera pas décrit davantage.

Chacune des parois latérales 119 et 120 délimite ainsi avec la paroi médiane 115 une cavité interne respective 124, 125 destinée à recevoir des objets.

Si les deux cavités 124 et 125 ne sont pas ainsi remplies intégralement d'objets, le sac 114 est difforme, inesthétique, et les objets qui y sont éventuellement placés peuvent bouger, s'entrechoquer, au risque de se détériorer.

Un dispositif conforme à la présente invention, logé respectivement dans chacune des cavités 124 et 125, et désigné respectivement par la référence 126 ou 127, permet d'éviter ces inconvénients.

A cet effet, la coque 1 de chacun des dispositifs selon l'invention 126, 127 épouse par sa paroi 2 le contour interne, déterminé, de la paroi latérale respective 119, 120 occupant une conformation correspondant à celle qu'elle présenterait si la cavité respective 124, 125 était pleine, ce qui rigidifie cette paroi respective 119 , 120 dans une telle conformation.

Par contre, le volume interne 4 de chaque coque 1 est intégralement ouvert vers la paroi médiane 115 du sac 114, le long des bords de laquelle les deux parois 2 et 3 de chaque coque 1 se raccordent à cette paroi 155 par une lèvre périphérique respective 128.

Le long de cette lèvre 128 se raccorde également aux parois 2 et 3, de façon étanche, une paroi étanche, souple, élastiquement extensible 129 qui délimite ainsi avec la paroi 3 de l'enceinte étanche 5, dans le volume interne 4 du dispositif 126 ou 127 respectivement, une chambre étanche respective 130 accessible par une valve 131 traversant la paroi latérale respectivement correspondante 119, 120, de même que la valve 6 d'accès à l'enceinte étanche 5 de la coque 1.

Lorsque le sac 114 est vide, on peut comme on l'a illustré à propos de la cavité 124 placer la paroi 129 au contact de la paroi médiane 115 en ouvrant la chambre étanche 130 à l'air libre par la valve 131 et en laissant jouer l'élasticité de la paroi 129, ou en introduisant de l'air en légère surpression à l'intérieur de la chambre étanche 130 par la valve 131 ; si les deux cavités 124 et 125 sont vides d'objet à transporter, les deux dispositifs selon l'invention 126 et 127 peuvent être placés dans cet état.

Si, comme on l'a illustré à propos de la cavité 125, l'une des cavités 124 et 125, ou chacune de ces cavités, est destinée à être remplie partiellement d'objets à transporter tels que 132, ces objets sont placés au contact de la paroi médiane 115 dans cette cavité telle que 125, alors ouverte, puis on ferme cette cavité telle que 125 par les moyens 123 ; au cours de cette fermeture, la paroi 129 se déforme en venant au contact des objets 132 qu'elle tend à plaquer contre la paroi médiane 115, la valve 131 étant ouverte pour mettre la chambre étanche 130 à la pression atmosphérique ; ensuite, par la valve 131, on introduit dans la chambre étanche 130 de l'air en légère surpression par rapport à la pression atmosphérique, ce qui plaque étroitement la paroi 129 sur les objets 132 eux-mêmes en appui contre la paroi médiane 115, et cale ainsi ces objets à l'intérieur du sac 114 ; naturellement, on peut pratiquer de même à l'intérieur de la cavité 124.

L'élasticité de la paroi 129 est choisie telle que celle-ci puisse, le cas échéant, venir se plaquer contre la paroi 3 de l'enceinte 5 de la coque 1 si la cavité correspondante 124 ou 125 doit être intégralement emplie d'objets 132 à transporter, la chambre correspondante 130 pouvant alors rester à la pression atmosphérique.

Lorsqu'on désire reprendre les objets 132 ainsi logés dans le sac 114, on rétablit par la valve 131 la pression atmosphérique dans la chambre 130 correspondante puis on ouvre le sac 114 pour reprendre les objets 132.

Si l'on désire extraire le dispositif selon l'invention 126, 127 de la cavité correspondante 124, 125, on peut par la valve 106 établir la pression atmosphérique dans l'enceinte étanche 5, ce qui assouplit la coque 1 et permet par conséquent de réduire son encombrement pour la faire sortir du sac, puis éventuellement l'introduire à nouveau dans celui-ci ; alors, pour redonner à la coque 1 sa conformation, on laisse l'enceinte étanche 5 ouverte à l'air libre par la valve 6 et, après avoir fermé le sac 114, on établit une légère surpression dans la chambre 130, ce qui d'une part plaque la paroi 129 contre la paroi médiane 115 du sac 114 et d'autre part plaque la coque 1 contre la paroi latérale respective 119, 120 ; alors, on rigidifie la coque 1 dans la conformation obtenue en établissant une dépression relative dans l'enceinte étanche 5, puis on supprime la surpression dans la chambre 130 pour utiliser ensuite le sac 114 comme on l'a indiqué plus haut.

Naturellement, bien que le sac illustré à la figure 10 présente deux cavités 124 et 125 définies, respectivement de part et d'autre d'une paroi médiane rigide 115, par des parois latérales souples respectives 119, 120, on ne sortirait pas du cadre de la présente invention en prévoyant un sac comportant une paroi latérale souple unique, comparable à l'une des parois 119 et 120, délimitant une cavité avec une paroi rigide comparable à la paroi 115 mais également latérale, c'est-à-dire délimitant ladite cavité vers l'extérieur du sac, et en prévoyant à l'intérieur de cette cavité un dispositif conforme à la présente invention, c'est-à-dire comparable à l'un des dispositifs 126 et 127, et coopérant avec ladite paroi rigide et avec ladite paroi souple comme chaque dispositif 126, 127 coopère avec la paroi 115 et, respectivement, l'une ou l'autre des parois 119, 120.

La figure 11 illustre précisément un sac à dos 162 présentant une telle conception, c'est-à-dire comportant une paroi rigide ou semi-rigide 163, de forme générale anatomiquement adaptée à un appui sur le dos d'un utilisateur par une face 164, et une paroi souple enveloppante 165 délimitant avec une autre face 166 de la paroi 163, à l'opposé de la face 164, une cavité 167 à la façon dont l'une quelconque des parois souples 119, 120 du sac de voyage 114 illustré à la figure 10 délimite une cavité 124, 125 avec la paroi 115 ; à cet effet, les parois 163 et 165 présentent une périphérie commune 168 sur la majeure partie de laquelle elles sont solidarisées mutuellement de façon continue, en permanence, et sur une faible partie, par exemple supérieure, de laquelle elles sont solidarisées mutuellement par des moyens 169, tels que des boucles et des sangles, permettant d'ouvrir à volonté la cavité 167 pour y loger ou y prendre des objets 170 qu'elle est destinée à contenir, et de la refermer à volonté, à la façon de l'une quelconque des cavités 124, 125 du sac 114 à la description auquel on se réfèrera à cet égard.

Des sangles telles que 180 solidaires de la paroi 163 et placées en regard de la face 164 de celle-ci permettent d'accrocher le sac 162 aux épaules de l'utilisateur , comme il est connu en soi dans le domaine des sacs à dos.

Pour caler contre la face 166 de la paroi 163 des objets 170 n'occupant pas intégralement la cavité 167 et donner une forme régulière à la paroi souple 165, la cavité 167 loge un dispositif selon l'invention 171 en tout point analogue aux dispositifs 126, 127 précédemment décrits notamment quant au mode de coopération de sa coque 1 avec la paroi souple 165, analogue au mode de coopération des coques 1 des dispositifs 126 et 127 avec les parois souples 119 et 120 respectivement, et quant au mode de coopération de sa paroi 172, correspondant à la paroi 129 des dispositifs 126 et 127 et délimitant comme ces dernières avec la coque 1 une chambre étanche 173 susceptible d'être gonflée ou dégonflée par l'intermédiaire d'une valve 174 accessible de l'extérieur du sac 162, avec la face 166 de la paroi 163 et avec les objets 170 éventuels.

Naturellement, le sac à dos 162 peut présenter toute forme d'ensemble désirée, et toute disposition propre à en augmenter la commodité et le confort d'utilisation ; à titre d'exemple non limitatif, il peut ainsi comporter sur la face 164 de la paroi 163, de façon solidaire de celle-ci, un coussin élastiquement compressible 175, par exemple réalisé sous forme d'une chambre étanche 176 dé!imitée par une paroi souple 177 et étanche solidarisée périphériquement, de façon continue et étanche, avec la paroi 163 alors également étanche, cette chambre 176 étant gonflable et dégonflable par l'intermédiaire d'une valve 178, et sur ce coussin 175 un revêtement par exemple textile 179 destiné à venir au contact du dos de l'utilisateur et facilitant une circulation d'air entre ce dos et le coussin 175.

Un Homme du métier comprendra aisément que les dispositifs 126, 127, 171 qui viennent d'être décrits peuvent également équiper des cavités autres que les cavités internes d'un sac souple 114 ou 162, et notamment des cavités délimitées par des parois rigides dans lesquelles les dispositifs selon l'invention ont pour seule fonction de caler des objets n'occupant pas l'intégralité de ces cavités ; on peut citer, à titre d'exemples non limitatifs, les coffres à bagages de véhicules automobiles, de bateaux ou d'avions, notamment petit ou moyen courrier, les soutes et cales de bateaux, les conteneurs....

## Revendications

1. Dispositif du type comportant une coque rigide enveloppante (1), délimitant un volume interne (4) et constituée de parois étanches (2, 3), souples, délimitant entre elles une enceinte étanche (5) placée en dépression par rapport à une pression ambiante, et d'un matériau de remplissage (7) emplissant intégralement ladite enceinte étanche (5) placée en dépression, ledit matériau de remplissage (7) étant d'un type se présentant à l'état rigide dans ladite enceinte étanche (5) placée en dépression et susceptible de se présenter à l'état souple lorsqu'il est placé à la pression ambiante,
caractérisé en ce qu'il comporte au moins une chambre étanche intérieure (4, 12 à 14, 44, 61 à 63, 81, 82, 105 à 108, 130, 150, 173) occupant ledit volume interne (4).

2. Dispositif selon la revendication 1, caractérisé en ce que ledit matériau de remplissage (7) est un matériau granulaire (8) retenu par un réseau (9) perméable aux gaz mais imperméable audit matériau granulaire (8).

3. Dispositif selon l'une quelconque des revendications 1 et 2, caractérisé en ce qu'il comporte des moyens (6) formant valve d'accès à l'intérieur de ladite enceinte étanche (5) placée en dépression.

4. Dispositif selon l'une quelconque des revendications 1 à 3, caractérisé en ce qu'il comporte ladite chambre étanche intérieure (4, 12 à 14, 44, 61 à 63, 81, 82, 105 à 108, 130, 150) en plusieurs exemplaires (12 à 14, 44, 61 à 63, 81, 82, 105 à 108, 150) occupant conjointement ledit volume interne (7).

5. Dispositif selon l'une quelconque des revendications 1 à 4, caractérisé en ce que ladite chambre étanche intérieure (12 à 14, 44, 61 à 63, 81, 82, 105 à 108, 150), respectivement au moins l'un desdits exemplaires (12 à 14, 44, 61 à 63, 81, 82, 105 à 108, 150), est dissociable de ladite coque rigide (1).

6. Dispositif selon l'une quelconque des revendications 1 à 5, caractérisé en ce que ladite chambre étanche intérieure (4, 12 à 14, 44, 61 à 63, 81, 82, 105 à 108, 130, 150, 173), respectivement aumoins l'un desdits exemplaires (12 à 14, 44, 61 à 63, 81, 82, 105 à 108, 150), contient un matériau fluide choisi dans un groupe comportant les gaz, les gels, les liquides, les matériaux particulaires et/ou un matériau expansé.

7. Dispositif selon la revendication 6, caractérisé en ce que ledit matériau fluide est placé à une pression sensiblement identique à la pression ambiante.

8. Dispositif selon la revendication 6, caractérisé en ce que ledit matériau fluide est placé à une pression sensiblement supérieure à la pression ambiante.

9. Dispositif selon l'une quelconque des revendications 6 à 8, caractérisé en ce qu'il comporte des moyens (69) pour faire circuler ledit matériau fluide.

10. Dispositif selon l'une quelconque des revendications 1 à 9, caractérisé en ce qu'il comporte des moyens (18, 35, 42, 45, 131, 156, 174) formant valve d'accès à l'intérieur de ladite chambre étanche (4, 12 à 14, 44, 61 à 63, 81, 82, 105 à 108, 130, 150, 173), respectivement d'au moins l'un desdits exemplaires (12 à 14, 44, 61 à 63, 81, 82, 105 à 108, 150).

11. Dispositif selon l'une quelconque des revendications 1 à 10, caractérisé en ce qu'il comporte une housse souple (133, 134, 137, 138, 151, 157) enveloppant ladite coque rigide (1) à l'extérieur dudit volume interne (4).

12. Dispositif selon l'une quelconque des revendications 1 à 11, caractérisé en ce que ladite coque rigide (1) enveloppe intégralement, de façon continue, ledit volume interne (4).

13. Dispositif selon l'une quelconque des revendications 1 à 12, caractérisé en ce que ladite coque rigide (1) présente des lèvres (25, 26, 59, 60, 75, 76, 92 à 95, 128, 154) délimitant une discontinuité localisée de ladite coque rigide (1).

14. Dispositif selon la revendication 13, caractérisé en ce que lesdites lèvres (59, 60) sont mutuellement jointives et en ce que ladite coque rigide (1) enveloppe intégralement ledit volume interne (4).

15. Dispositif selon la revendication 14, caractérisé en ce qu'il comporte des moyens (137) d'assemblage mutuel desdites lèvres (59, 60).

16. Dispositif selon la revendication 15, caractérisé en ce que lesdits moyens d'assemblage mutuel sont portés par lesdites lèvres (59, 60).

17. Dispositif selon la revendication 15 en combinaison avec la revendication 11, caractérisé en ce que lesdits moyens d'assemblage mutuel (137) sont portés et/ou constitués par ladite housse (137).

18. Dispositif selon la revendication 13, caractérisé en ce que lesdites lèvres (25, 26, 75, 76, 92 à 95, 128, 154)) sont mutuellement espacées et en ce que ladite coque rigide (1) enveloppe partiellement, de façon discontinue, ledit volume interne (4).

19. Dispositif selon la revendication 18, caractérisé en ce qu'il comporte des moyens (28, 41, 78, 79, 96, 97, 138, 141, 142, 155) de liaison mutuelle desdites lèvres mutuellement espacées (25, 26, 39, 75, 76, 92 à 95).

20. Dispositif selon la revendication 19, caractérisé en ce que lesdits moyens (78, 79, 96, 97, 138, 141, 142) de liaison mutuelle sont élastiquement déformables en extension entre lesdites lèvres.

21. Dispositif selon l'une quelconque des revendications 19 et 20, caractérisé en ce que lesdits moyens (28, 41, 155) de liaison mutuelle sont élastiquement déformables en torsion et/ou flexion entre lesdites lèvres (25, 26, 39).

22. Dispositif selon l'une quelconque des revendications 19 à 21, caractérisé en ce que lesdits moyens (28, 41) de liaison mutuelle sont élastiquement déformables en compression.

23. Dispositif selon l'une quelconque des revendications 19 à 22, caractérisé en ce que lesdits moyens (28, 41, 78, 79, 96, 97, 155) de liaison mutuelle comportent au moins un lien (78, 79, 96, 97) reliant localement lesdites lèvres (75, 76, 92 à 95).

24. Dispositif selon l'une quelconque des revendications 19 à 22, caractérisé en ce que lesdits moyens (28, 41, 78, 79, 96, 97, 155) de liaison mutuelle comportent une paroi (28, 41, 155) complétant ladite coque rigide (1) pour délimiter ledit volume interne (4) de façon continue.

25. Dispositif selon l'une quelconque des revendications 19 à 24, caractérisé en ce que lesdits moyens (28, 41, 78, 79, 96, 97, 155) de liaison mutuelle sont portés par lesdites lèvres (25, 26, 39, 75, 76, 92 à 95).

26. Dispositif selon l'une quelconque des revendications 19 à 20 en combinaison avec la revendication 11, caractérisé en ce que lesdits moyens de liaison mutuelle (138, 141, 142) sont portés et/ou constitués par ladite housse (138).

27. Dispositif selon l'une quelconque des revendications 1 à 26, caractérisé en ce qu'il comporte des moyens (86, 158) définissant un relief déterminé à l'intérieur dudit volume interne (4).

28. Dispositif selon la revendication 27 en combinaison avec l'une quelconque des revendications 19 à 26, caractérisé en ce que lesdits moyens (17, 41, 155) de liaison mutuelle présentent au moins une protubérance localisée (86, 136, 158) à l'intérieur dudit volume interne (4).

29. Dispositif selon l'une quelconque des revendications 27 et 28, caractérisé en ce que ladite protubérance localisée (86) est amovible.

30. Dispositif selon l'une quelconque des revendications 1 à 29, caractérisé en ce qu'il comporte des moyens (29, 48, 133) de protection à l'encontre des chocs et/ou des frottements accolés à ladite coque rigide (1) à l'extérieur dudit volume interne (4).

31. Dispositif selon la revendication 30, caractérisé en ce que lesdits moyens (29, 48) de protection sont localisés.

32. Dispositif selon l'une quelconque des revendications 30 et 31, caractérisé en ce que lesdits moyens (29, 48) de protection sont portés par ladite coque rigide (1).

33. Dispositif selon l'une quelconque des revendications 30 et 31 en combinaison avec la revendication 11, caractérisé en ce que lesdits moyens de protection (29, 133) sont portés et/ou constitués par ladite housse (133, 134).

34. Dispositif selon l'une quelconque des revendications 1 à 33, caractérisé en ce qu'il comporte au moins une chambre étanche extérieure (32, 52, 143, 145) accolée à ladite coque rigide (1) à l'extérieur dudit volume interne (4) et contenant un matériau fluide choisi dans un groupe comportant les gaz, les gels, les liquides, les matériaux particulaires.

35. Dispositif selon la revendication 34, caractérisé en ce que ledit matériau fluide est placé à une pression sensiblement identique à la pression ambiante.

36. Dispositif selon la revendication 34, caractérisé en ce que ledit matériau fluide est placé à une pression sensiblement supérieure à la pression ambiante.

37. Dispositif selon l'une quelconque des revendications 34 à 36, caractérisé en ce qu'il comporte des moyens (69) pour faire circuler ledit matériau fluide.

38. Dispositif selon l'une quelconque des revendications 34 à 36, caractérisé en ce qu'il comporte ladite chambre extérieure en plusieurs exemplaires (145) et en ce que des moyens (148) sont prévus pour établir de façon prédéterminée des pressions respectives prédéterminées, variables dans chacun desdits exemplaires (145).

39. Dispositif selon l'une quelconque des revendications 34 à 38, caractérisé en ce que ladite chambre étanche extérieure (32, 52), respectivement chaque exemplaire de celle-ci, est portée par ladite coque rigide (1).

40. Dispositif selon l'une quelconque des revendications 34 à 38 en combinaison avec la revendication 11, caractérisé en ce que ladite chambre extérieure, respectivement chaque exemplaire (145) de celle-ci, est portée et/ou constituée par ladite housse (138).

41. Dispositif selon l'une quelconque des revendications 1 à 40, caractérisé en ce qu'il consiste en un réservoir (fig. 1).

42. Dispositif selon la revendication 41 en combinaison avec les revendications 4 et 6, caractérisé en ce qu'il comporte des moyens (21, 22) pour, en alternance, introduire un premier fluide à stocker dans un premier (12) desdits exemplaires en vidant un second (13, 14) desdits exemplaires et introduire un deuxième fluide de commande dans ledit second exemplaire (13, 14) en chassant le fluide à stocker dudit premier exemplaire (12).

43. Dispositif selon l'une quelconque des revendications 1 à 40, caractérisé en ce qu'il consiste en un dispositif flottant et/ou glissant choisi dans un groupe comportant les flotteurs, coques de bâteau, planches à voile, luges, skis (fig. 3, 4).

44. Dispositif selon la revendication 43 en combinaison avec la revendication 24, caractérisé en ce que ladite paroi (28, 41) complétant ladite coque rigide (1) constitue une paroi inférieure du dispositif.

45. Dispositif selon l'une quelconque des revendications 1 à 40, caractérisé en ce qu'il consiste en un dispositif de calage et/ou de soutien du corps humain choisi dans un groupe comportant les matelas ou coussins anti-escarres et les matelas chirurgicaux (fig. 5 à 9).

46. Dispositif selon la revendication 45, caractérisé en ce que ladite coque rigide (1) présente une zone supérieure (54, 72, 89) de forme générale plate, une zone inférieure (55, 73, 90) de forme générale plate, et une zone périphérique (58, 74, 91) en forme de jupe reliant mutuellement lesdites zones supérieure et inférieure (54, 72, 89, 55, 73, 90) de formes générales respectives plates.

47. Dispositif selon la revendication 46 en combinaison avec la revendication 13, caractérisé en ce que ladite zone inférieure (51, 73, 90) est discontinue et présente lesdites lèvres (59, 60, 75, 76, 92 à 95).

48. Dispositif selon l'une quelconque des revendications 46 et 47 en combinaison avec la revendication 4, caractérisé en ce qu'au moins certains (62, 63, 81, 82, 105, 106) desdits exemplaires présentent la forme de plots mutuellement juxtaposés sous ladite zone supérieure (54, 72, 89).

49. Dispositif selon la revendication 48 ou l'une quelconque des revendications 46 et 47 en combinaison avec la revendication 4, caractérisé en ce qu'au moins certains (62, 63, 81, 82, 105, 106) desdits exemplaires présentent la forme de boudins de même direction longitudinale déterminée, mutuellement juxtaposés transversalement à ladite direction, sous ladite zone supérieure (54, 72, 89).

50. Dispositif selon la revendication 49, caractérisé en ce que d'autres (107, 108) desdits exemplaires présentent la forme de boudins de même direction transversale, lesdits boudins de même direction longitudinale déterminée et lesdits boudins de même direction transversale étant mutuellement superposés sous ladite zone supérieure (89).

51. Dispositif selon l'une quelconque des revendications 46 et 47 en combinaison avec la revendication 4 ou des revendications 48 à 50, caractérisé en ce qu'il comporte des moyens (70, 85, 113) pour provoquer de façon commandée une succession d'étapes consistant à :
- placer ladite enceinte étanche (5) à la pression ambiante,
- gonfler ou dégonfler de façon déterminée certains, déterminés, desdits exemplaires,
- replacer ladite enceinte étanche (5) en dépression par rapport à la pression ambiante.

52. Dispositif selon l'une quelconque des revendications 46 à 51, caractérisé en ce que lesdites parois étanches sont comparativement inextensibles dans ladite zone périphérique et comparativement élastiquement extensibles dans ladite zone supérieure.

53. Dispositif selon l'une quelconque des revendications 46 à 52 en combinaison avec la revendication 11, caractérisé en ce que ladite housse est comparativement inextensible dans une zone correspondant à ladite zone périphérique et comparativement élastiquement extensible dans une zone correspondant à ladite zone supérieure.

54. Dispositif selon l'une quelconque des revendications 1 à 40, caractérisé en ce qu'il consiste en un dispositif (126, 127, 173) de calage et/ou de soutien d'au moins un objet (132, 170) dans une cavité (124, 125, 167) présentant un contour interne, telle qu'un bagage, un conteneur, un coffre de véhicule ou analogue, ladite coque rigide enveloppante (1) épousant ledit contour interne à l'opposé dudit volume interne (4).

## Claims

1. A device of the type having a rigid surrounding shell (1), delimiting an internal volume (4) and constituted by flexible fluid tight walls (2, 3) delimiting between them a fluid tight enclosure (5) held at a pressure below ambient pressure, and by a filling material (7) completely filling the said fluid tight enclosure (5) held below ambient pressure, the said filling material (7) being of a type having a rigid state in the said fluid tight enclosure (5) held below ambient pressure and able to have a flexible state when it is at ambient pressure,
characterised in that it comprises at least one internal fluid tight chamber (4, 12 to 14, 44, 61 to 63, 81, 82, 105 to 108, 130, 150, 173) occupying the said internal volume (4).

2. A device according to Claim 11 characterised in that the said filling material (7) is a granular material (8) hold by an array (9) permeable to gases but impermeable to the said granular material (8).

3. A device according to either of Claims 1 and 2, characterised in that it comprises means (6) forming a valve for access to the interior of the said fluid tight enclosure (5) held below ambient pressure.

4. A device according to any one of Claims 1 to 3, characterised in that it comprises the said interior fluid tight chamber (4, 12 to 14, 44, 61 to 63, 81, 82, 105 to 108, 130, 150) in several examples (12 to 14, 44, 61 to 63, 81, 82, 105 to 108, 150) together occupying the said internal volume (7).

5. A device according to any of Claims 1 to 4, characterised in that the said fluid tight internal chamber (12 to 14, 44, 61 to 63, 81, 82, 105 to 108, 150), respectively at least one of the said examples (12 to 14, 44, 61 to 63, 81, 82, 105 to 108, 150), is removable from the said rigid shell (1).

6. A device according to any one of Claims 1 to 5 characterised in that the said internal fluid tight chamber (4, 12 to 14, 44, 61 to 63, 81, 82, 105 to 108, 130, 150, 173), respectively at least one of the said examples (12 to 14, 44, 61 to 63, 81, 82, 105 to 108, 150), contains a fluid material chosen within a group comprising gasses, gels, liquids, particulate materials and/or an expanded material.

7. A device according to Claim 6, characterised in that the said fluid material is placed at a pressure substantially identical to ambient pressure.

8. A device according to Claim 6, characterised in that the said fluid material is placed at a pressure substantially greater than ambient pressure.

9. A device according to any one of Claims 6 to 8, characterised in that it comprises means (69) for circulating the said fluid material.

10. A device according to any one of Claims 1 to 9, characterised in that it comprises means (18, 35, 42, 45, 131, 156, 174) forming a valve for access to the interior of the said fluid tight chamber (4, 12 to 14, 44, 61 to 63, 81, 82, 105 to 108, 130, 150, 173), respectively of at least one of the said examples (12 to 14, 44, 61 to 63, 81, 82, 105 to 108, 150).

11. A device according to any one of Claims 1 to 10, characterised in that it comprises a flexible cover (133, 134, 137, 138, 151, 157) enclosing the said rigid shell (1) outside the said internal volume (4).

12. A device according to any one of Claims 1 to 11, characterised in that the said rigid shell (1) entirely surrounds, in a continuous manner, the said internal volume (4).

13. A device according to any one of Claims 1 to 12, characterised in that the said rigid shell (1) has edges (25, 26, 59, 60, 75, 76, 92 to 95, 128, 154) delimiting a localised discontinuity of the said rigid shell (1).

14. A device according to Claim 13, characterised in that the said edges (59, 60) are mutually adjoint and in that the said rigid shell (1) entirely surrounds the said internal volume (4).

15. A device according to Claim 14, characterised in that it comprises means (137) for mutual assembly of the said edges (59, 60).

16. A device according to Claim 15, characterised in that the said means for mutual assembly are carried by the said edges (59, 60).

17. A device according to Claim 15, in combination with Claim 11 characterised in that the said means for mutual assembly (137) are carried and/or constituted by the said cover (137).

18. A device according to Claim 13, characterised in that the said edges (25, 26, 75, 76, 92 to 95, 128, 154) are mutually spaced and in that the said rigid shell (1) partially surrounds, in a discontinuous manner, the said internal volume (4).

19. A device according to Claim 18, characterised in that it comprises means (28, 41, 78, 79, 96, 97, 138, 141, 142, 155) for mutual connection of the said mutually spaced edges (25, 26, 39, 75, 76, 92 to 95).

20. A device according to Claim 19, characterised in that the said means (78, 79, 96, 97, 138, 141, 142) for mutual connection are elastically deformable in extension between the said edges.

21. A device according to either of Claims 19 and 20, characterised in that the said means (28, 41, 55) for mutual connection are elastically deformable in torsion and/or flexure between the said edges (25, 26, 39).

22. A device according to any one of Claims 19 to 21, characterised in that the said means (28, 41) for mutual connection are elastically deformable in compression.

23. A device according to any one of Claims 19 to 22, characterised in that the said means (28, 41, 78, 79, 96, 97, 155) for mutual connection have at least one connection (78, 79, 96, 97) locally connecting the said edges (75, 76, 92 to 95).

24. A device according to any one of Claims 19 to 22, characterised in that the said means (28, 41, 78, 79, 96, 97, 155) for mutual connection comprise a wall (28, 41, 155) complementing the said rigid shell (1) for delimiting the said internal volume (4) in a continuous manner.

25. A device according to any one of Claims 19 to 24, characterised in that the said means (28, 41, 78, 79, 96, 97, 155) for mutual connection are carried by the said edges (25, 26, 39, 75, 76, 92 to 95).

26. A device according to either of Claims 19 and 20 in combination with Claim 11, characterised in that the said means for mutual connection (138, 141, 142) are carried and/or constituted by the said cover (138).

27. A device according to any one of Claims 1 to 26, characterised in that it comprises means (86, 158) defining a determined relief inside the said internal volume (4).

28. A device according to Claim 27 in combination with any one of Claims 19 to 26, characterised in that the said means (17, 41, 155) for mutual connection have at least one localised projection (86, 136, 158) inside the said internal volume (4).

29. A device according to either of Claims 27 and 28, characterised in that the said localised projection (86) is removable.

30. A device according to any one of Claims 1 to 29, characterised in that it comprises means (29, 48, 133) for protection against shocks and/or frettings adhered to the said rigid shell (1) outside the said internal volume (4).

31. A device according to Claim 30, characterised in that the said protection means (29, 48) are localised.

32. A device according to either of Claims 30 and 31, characterised in that the said protection means (29, 48) are carried by the said rigid shell (1).

33. A device according to either of Claims 30 and 31 in combination with Claim 11, characterised in that the said protection means (29, 133) are carried by and/or constituted by the said cover (133, 134).

34. A device according to any one of Claims 1 to 33, characterised in that it comprises at least one external fluid tight chamber (32, 52, 143, 145) adhered to the said rigid shell (1) outside the said internal volume (4) and containing a fluid material chosen within a group comprising gasses, gels, liquids, particulate materials.

35. A device according to Claim 34, characterised in that the said fluid material is placed at a pressure substantially identical to ambient pressure.

36. A device according to Claim 34, characterised in that the said fluid material is placed at a pressure substantially greater than ambient pressure.

37. A device according to any one of Claims 34 to 36, characterised in that it comprises means (69) for circulating the said fluid material.

38. A device according to any one of Claims 34 to 36, characterised in that it comprises the said external chamber in several examples (145) and that means (148) are provided for establishing in a predetermined manner respective variable predetermined pressures in each of the said examples (145).

39. A device according to any one of Claims 34 to 38, characterised in that the said external fluid tight chamber (32, 52), respectively each example of this, is carried by the said rigid shell (1).

40. A device according to any one of Claims 34 to 38 in combination with Claim 11, characterised in that the said external chamber, respectively each example (145) of this, is carried and/or constituted by the said cover (138).

41. A device according to any one of Claims 1 to 40, characterised in that it consists of a reservoir (Figure 1).

42. A device according to Claim 41 in combination with Claims 4 and 6, characterised in that it comprises means (21, 22) for, alternatively, introducing a first fluid to be stored into a first (12) of the said examples whilst emptying a second (13, 14) of the said examples and introducing a second control fluid into the said second example (13, 14) chasing the said fluid to be stored from the said first example (12).

43. A device according to any one of Claims 1 to 40, characterised in that it consists of a floating and/or sliding device chosen within a group comprising floats, hulls of boats, sail boards, toboggans, skis (Figures 3, 4).

44. A device according to Claim 43 in combination with Claim 24, characterised in that the said wall (28, 41) complementing the said rigid shell (1) constitutes a lower wall of the device.

45. A device according to any one of Claims 1 to 40, characterised in that it consists of a device for holding and/or support of the human body chosen within a group comprising anti-bed sore mattresses or cushions and surgical mattresses (Figures 5 to 9).

46. A device according to Claim 45, characterised in that the said rigid shell (1) has an upper zone (54, 72, 89) of generally flat shape, a lower zone (55, 73, 90) of generally flat shape and a peripheral zone (58, 74, 91) in the form of a skirt mutually connecting the said upper and lower zones (54, 72, 89, 55, 73, 90) respectively of generally flat shape.

47. A device according to Claim 46 in combination with Claim 13, characterised in that the said lower zone (51, 73, 90) is discontinuous and has the said edges (59, 60, 75, 76, 90 to 95).

48. A device according to either of Claims 46 and 47 in combination with Claim 4, characterized in that at least certain (62, 63, 81, 82, 105, 106) of the said examples have the shape of studs mutually juxtaposed under the said upper zone (54, 72, 89).

49. A device according to Claim 48 or either of Claims 46 and 47 in combination with Claim 4, characterised in that at least certain (62, 63, 81, 82, 105, 106) of the said examples have the shape of tubes of the same predetermined longitudinal direction mutually juxtaposed transversely of the said direction, under the said upper zone (54, 72, 89).

50. A device according to Claim 49, characterised in that others (107, 108) of the said examples have the shape of tubes of the same transverse direction, the said tubes of the same longitudinal direction and the said tubes of the same transverse direction being mutually superimposed under the said upper zone (89).

51. A device according to either of Claims 46 and 47 in combination with Claim 4 or to any one of Claims 48 to 50, characterised in that it comprises means (70, 85, 113) for causing in a controlled manner a succession of stages consisting in:
- placing the said fluid tight enclosure (5) at ambient pressure,
- inflating or deflating in a predetermined manner certain, predetermined ones of the said examples,
- re-placing the said fluid tight enclosure (5) at pressure below ambient.

52. A device according to any one of Claims 46 to 51, characterised in that the said fluid tight walls are comparatively inextensible in the said peripheral zone and comparatively elastically extensible in the said upper zone.

53. A device according to any one of Claims 46 to 52 in combination with Claim 11, characterised in that the said cover is comparatively inextensible in a zone corresponding to the said peripheral zone and comparatively elastically extensible in a zone corresponding to the said upper zone.

54. A device according to any one of Claims 1 to 40, characterised in that it consists of a device (126, 127, 173) for holding and/or support of at least one object (132, 170) in a space (124, 125, 167) having an internal contour, such as a piece of baggage, a container, the luggage space of an automobile or the like, the said surrounding rigid shell (1) taking the said internal shape outside the said internal volume (4).

## Patentansprüche

1. Vorrichtung mit einer starren, einhüllenden Schale (1), welche ein inneres Volumen (4) eingrenzt und dichte Wände (2, 3) bildet, die zwischen sich einen dichten Zwischenraum (5) bilden, welcher bezüglich eines Umgebungsdruckes auf Unterdruck gehalten ist und ein Füllmaterial (7) enthält, das den genannten Zwischenraum (5) integral ausfüllt, wobei das Füllmaterial (7) solcher Art ist, daß es sich in dem Zwischenraum (5) bei dem Unterdruck in Starrem Zustand befindet, während es sich bei dem Umgebungsdruck in nachgiebigem Zustand befindet, dadurch gekennzeichnet, daß die Vorrichtung mindestens eine innere Kammer (4, 12-14, 44, 61-63, 81, 82, 105-108, 130, 150, 173) enthält, welche das innere Volumen (4) einnimmt.

2. Vorrichtung nach Anspruch 1, **dadurch gekennzeichnet,** daß das Füllmaterial (7) ein körniges Material (8) ist, welches von einem Netz (9) gehalten wird, das gasdurchlässig, jedoch undurchlässig für das körnige Material (8) ist.

3. Vorrichtung nach Anspruch 1 oder 2, **dadurch gekennzeichnet,** daß sie Mittel (6) aufweist, die ein Einlaßventil zum Inneren des unter Unterdruck stehenden dichten Zwischenraumes (5) bilden.

4. Vorrichtung nach einem der Ansprüche 1 bis 3, **dadurch gekennzeichnet,** daß die innere dichte Kammer (4, 12-14, 44, 61-63, 81, 82, 105-108, 130, 150) mehrere Abteile (12-14, 44, 61-63, 81, 82, 105-108, 150) bildet, welche gemeinsam das genannte innere Volumen (7) einnehmen.

5. Vorrichtung nach einem der Ansprüche 1 bis 4, **dadurch gekennzeichnet,** daß die innere dichte Kammer (12-14, 44, 61-63, 81, 82, 105-108, 150) bzw. mindestens eines der besagten Abteile (12-14, 44, 61-63, 81, 82, 105-108, 150) von der starren Schale (1) trennbar ist.

6. Vorrichtung nach einem der Ansprüche 1 bis 5, **dadurch gekennzeichnet,** daß die dichte innere Kammer (4, 12-14, 44, 61-63, 81, 82, 105-108, 130, 150, 173) bzw. mindestens eines der besagten Abteile (12-14, 44, 61-63, 81, 82, 105-108, 150) ein Fluid aus der Gruppe enthaltend Gase, Gele, Flüssigkeiten, teilchenförmige Stoffe und/oder einen expandierten Stoff aufweist.

7. Vorrichtung nach Anspruch 6, **dadurch gekennzeichnet,** daß das Fluid unter einem im wesentlichen dem Umgebungsdruck entsprechenden Druck steht.

8. Vorrichtung nach Anspruch 6, **dadurch gekennzeichnet,** daß das Fluid im wesentlichen unter einem oberhalb des Umgebungsdruckes liegenden Druck steht.

9. Vorrichtung nach einem der Ansprüche 6 bis 8, **dadurch gekennzeichnet,** daß sie Mittel (69) zum Zirkulieren des Fluids aufweist.

10. Vorrichtung nach einem der Ansprüche 1 bis 9, **dadurch gekennzeichnet,** daß sie Mittel (18, 35, 42, 45, 131, 156, 174) umfaßt, welche ein Einlaßventil zum Inneren der dichten Kammer (4, 12-14, 44, 61-63, 81, 82, 105-108, 130, 150, 173) bzw. mindestens zu einem der Abteile (12-14, 44, 61-63, 81, 82, 105-108, 150) bilden.

11. Vorrichtung nach einem der Ansprüche 1 bis 10, **dadurch gekennzeichnet,** daß sie einen schmiegsamen Überzug (133, 134, 137, 138, 151, 157) aufweist, welcher die Schale (1) außerhalb des besagten inneren Volumens (4) einhüllt.

12. Vorrichtung nach einem der Ansprüche 1 bis 11, **dadurch gekennzeichnet,** daß die starre Schale (1) das innere Volumen (4) kontinuierlich integral einschließt.

13. Vorrichtung nach einem der Ansprüche 1 bis 12, **dadurch gekennzeichnet,** daß die starre Schale (1) Lippen (25, 26, 59, 60, 75, 76, 92-95, 128, 154) bildet, die eine lokale Diskontinuität der starren Schale (1) begrenzen.

14. Vorrichtung nach Anspruch 13, **dadurch gekennzeichnet,** daß die Lippen (59, 60) gegenseitig anstoßen und daß die starre Schale (1) das innere Volumen (4) integral einschließt.

15. Vorrichtung nach Anspruch 14, **dadurch gekennzeichnet,** daß sie Mittel (137) zur gegenseitigen Anordnung der Lippen (59, 60) aufweist.

16. Vorrichtung nach Anspruch 15, **dadurch gekennzeichnet,** daß die genannten Anordnungsmittel von den Lippen (59, 60) getragen sind.

17. Vorrichtung nach Anspruch 15 in Verbindung mit Anspruch 11**, dadurch gekennzeichnet,** daß die Anordnungmittel (137) von dem Überzug (137) getragen und/oder gebildet sind.

18. Vorrichtung nach Anspruch 13, **dadurch gekennzeichnet,** daß die Lippen (25, 26, 75, 76, 92-95, 128, 154) gegenseitigen Abstand haben und daß die starre Schale (1) das innere Volumen (4) zum Teil diskontinuierlich einschließt.

19. Vorrichtung nach Anspruch 18**, dadurch gekennzeichnet,** daß sie Mittel (28, 41, 78, 79, 96, 97, 138, 141, 142, 155) zum gegenseitigen Verbinden der voneinander im Abstand befindlichen Lippen (25, 26, 39, 75, 76, 92-95) aufweist.

20. Vorrichtung nach Anspruch 19, **dadurch gekennzeichnet,** daß die genannten Mittel (78, 79, 96, 97, 138, 141, 142) zum gegenseitigen Verbinden elastisch dehnverformbar zwischen den Lippen gespannt sind.

21. Vorrichtung nach Anspruch 19 oder 20, **dadurch gekennzeichnet,** daß die genannten Mittel (28, 41, 155) zum gegenseitigen Verbinden elastisch torsions- und/oder biegeverformbar zwischen den Lippen (25, 26, 39) sind.

22. Vorrichtung nach einem der Ansprüche 19 bis 21, **dadurch gekennzeichnet,** daß die Mittel (28, 41) zum gegenseitigen Verbinden elastisch druckverformbar sind.

23. Vorrichtung nach einem der Ansprüche 19 bis 22, **dadurch gekennzeichnet,** daß die besagten Mittel (28, 41, 78, 79, 96, 97, 155) zum gegenseitigen Verbinden mindestens ein Band (78, 79, 96, 97) aufweisen, welches die Lippen (75, 76, 92-95) lokal verbindet.

24. Vorrichtung nach einem der Ansprüche 19 bis 22, **dadurch gekennzeichnet,** daß die Mittel (28, 41, 78, 79, 96, 97, 155) zum gegenseitigen Verbinden mindestens eine Wand (28, 41, 155) aufweist, welche die starre Schale (1) vervollständigt, um das innere Volumen (4) in kontinuierlicher Weise zu begrenzen.

25. Vorrichtung nach einem der Ansprüche 19 bis 24, **dadurch gekennzeichnet,** daß die Mittel (28, 41, 78, 79, 96, 97, 155) zum gegenseitigen Verbinden von den Lippen (25, 26, 39, 75, 76, 92-95) getragen sind.

26. Vorrichtung nach Anspruch 19 oder 20 in Verbindung mit Anspruch 11, **dadurch gekennzeichnet,** daß die Mittel zum gegenseitigen Verbinden (138, 141, 142) von dem besagten Überzug (138) getragen und/oder gebildet sind.

27. Vorrichtung nach einem der Ansprüche 1 bis 26, **dadurch gekennzeichnet,** daß sie Mittel (86, 158) umfaßt, welche ein vorgegebenes Relief bilden, das zum Inneren des inneren Volumens (4) hin begrenzt ist.

28. Vorrichtung nach Anspruch 27 in Verbindung mit einem der Ansprüche 19 bis 26, **dadurch gekennzeichnet,** daß die Mittel (17, 41, 155) zum gegenseitigen Verbinden mindestens einen örtlichen Vorsprung (86, 136, 158) nach innen in das innere Volumen (4) hinein bilden.

29. Vorrichtung nach Anspruch 27 oder 28, **dadurch gekennzeichnet,** daß der örtliche Vorsprung (86) beweglich ist.

30. Vorrichtung nach einem der Ansprüche 1 bis 29, **dadurch gekennzeichnet,** daß sie Schutzmittel (29, 48, 133) zum Schutz vor Stoß- und/oder Reibungsbelastungen aufweisen, welche auf die starre Schale (1) von außerhalb des inneren Volumens (4) ausgeübt werden.

31. Vorrichtung nach Anspruch 30, **dadurch gekennzeichnet,** daß die Schutzmittel (29, 48) lokalisiert sind.

32. Vorrichtung nach Anspruch 30 oder 31**, dadurch gekennzeichnet,** daß die Schutzmittel (29, 48) von der starren Schale (1) getragen sind.

33. Vorrichtung nach Anspruch 30 oder 31 in Verbindung mit Anspruch 11, **dadurch gekennzeichnet,** daß die Schutzmittel (29, 133) von dem Überzug (133, 134) getragen und/oder gebidet sind.

34. Vorrichtung nach einem der Ansprüche 1 bis 33, **dadurch gekennzeichnet,** daß sie mindestens eine äußere dichte Kammer (32, 52, 143, 145) aufweist, welche an die starre Schale (1) außerhalb des inneren Volumens (4) angefügt ist und ein Fluid aus der Gruppe enthaltend Gase, Gele, Flüssigkeiten, teilchenförmige Materialien enthält.

35. Vorrichtung nach Anspruch 34, **dadurch gekennzeichnet,** daß das Fluid einem Druck ausgesetzt ist, der im wesentlichen identisch mit dem Umgebungsdruck ist.

36. Vorrichtung nach Anspruch 34, **dadurch gekennzeichnet,** daß das Fluid einem Druck ausgesetzt ist, der im wesentlichen oberhalb des Umgebungsdruckes liegt.

37. Vorrichtung nach einem der Ansprüche 34 bis 36, **dadurch gekennzeichnet,** daß es Mittel (69) zum Zirkulierenlassen des Fluids aufweist.

38. Vorrichtung nach einem der Ansprüche 34 bis 36, **dadurch gekennzeichnet,** daß die äußere Kammer in mehrere Abteile (145) eingeteilt ist und daß Mittel (148) vorgesehen sind, um in vorbestimmter Weise jeweils vorbestimmte, variable Drucke in den einzelnen Abteilen (145) einzustellen.

39. Vorrichtung nach einem der Ansprüche 34 bis 38, **dadurch gekennzeichnet,** daß die äußere dichte Kammer (32, 52) bzw. jedes Anteil derselben von der starren Schale (1) getragen ist.

40. Vorrichtung nach einem der Ansprüche 34 bis 38 in Verbindung mit Anspruch 11, **dadurch gekennzeichnet,** daß die äußere Kammer bzw. jedes Anteil (145) derselben von dem Überzug (138) getragen und/oder gebildet ist.

41. Vorrichtung nach einem der Ansprüche 1 bis 40, **dadurch gekennzeichnet,** daß sie aus einem Reservoir besteht (Fig. 1).

42. Vorrichtung nach Anspruch 41 in Verbindung mit einem der Ansprüche 4 bis 6, **dadurch gekennzeichnet,** daß sie Mittel (21, 22) zum alternierenden Einführen eines ersten Fluids zum Lagern in einem ersten (12) der besagten Anteile durch Entleeren eines zweiten (13, 14) der besagten Anteile und zum Einführen eines zweiten Steuerfluids in das zweite Anteil (13, 14) zum Austreiben des zu lagernden Fluids aus dem ersten Anteil (12) aufweist.

43. Vorrichtung nach einem der Ansprüche 1 bis 40, **dadurch gekennzeichnet,** daß sie aus einer Schwimm- und/oder Gleitvorrichtung aus der Gruppe enthaltend Schwimmkörper, Bootsschalen, Segelbretter, Rodelschlitten, Ski besteht (Fig. 3, 4).

44. Vorrichtung nach Anspruch 43 in Verbindung mit Anspruch 24, **dadurch gekennzeichnet,** daß die besagte Wand (28, 41), welche die starre Schale (1) vervollständigt, eine Innenwand der Vorrichtung bildet.

45. Vorrichtung nach einem der Ansprüche 1 bis 40, **dadurch gekennzeichnet,** daß sie aus einer Einrichtung zur Einstellung und/oder Unterstützung des menschlichen Körpers aus der Gruppe enthaltend Matratzen oder Kissen, welche einem Wundliegen vorbeugen, und chirurgische Matratzen bestehen (Fig. 5 bis 9).

46. Vorrichtung nach Anspruch 45, **dadurch gekennzeichnet,** daß die starre Schale (1) eine obere Zone (54, 72, 89) von im wesentlichen ebener Gestalt, eine untere Zone (55, 73, 90) von im wesentlichen ebener Gestalt und eine Umfangszone (58, 74, 91) in Gestalt eines Rocks bildet, der die obere Zone und die untere Zone (54, 72, 89; 55, 73, 90) von im wesentlichen jeweils ebener Gestalt gegenseitig verbindet.

47. Vorrichtung nach Anspruch 46 in Verbindung mit Anspruch 13, **dadurch gekennzeichnet,** daß die untere Zone (51, 73, 90) diskontinuierlich ist und die besagten Lippen (59, 60, 75, 76, 92-95) hat.

48. Vorrichtung nach Anspruch 46 oder 47 in Verbindung mit Anspruch 4, **dadurch gekennzeichnet,** daß mindestens einige (62, 63, 81, 82, 105, 106) der Anteile die Gestalt von unter der oberen Zone (54, 72, 89) einander benachbarten Kästen bilden.

49. Vorrichtung nach Anspruch 48 oder Anspruch 46 oder 47 in Verbindung mit Anspruch 4, **dadurch gekennzeichnet,** daß mindestens einige (62, 63, 81, 82, 105, 106) der Anteile die Gestalt von Wülsten in gleicher Längsausrichtung aufweisen, die quer zu der genannten Richtung unterhalb der oberen Zone (54, 72, 89) nebeneinander angeordnet sind.

50. Vorrichtung nach Anspruch 49, **dadurch gekennzeichnet,** daß andere Anteile (107, 108) die Gestalt von Wülsten in gleicher Querausrichtung aufweisen, wobei die Wülste gleicher Längsausrichtung und die Wülste gleicher Querausrichtung unterhalb der oberen Zone (89) übereinandergelegt sind.

51. Vorrichtung nach Anspruch 46 oder 47 in Verbindung mit Anspruch 4 oder einem der Ansprüche 48 bis 50, **dadurch gekennzeichnet,** daß sie Mittel (70, 85, 113) zum gesteuerten Erzeugen einer Folge von folgenden Schritten aufweist:
- Bringen des dichten Zwischenraumes (5) auf Umgebungsdruck,
- Aufpumpen oder Druckablassen in vorbestimmter Weise von bestimmten Anteilen,
- Bringen des dichten Zwischenraumes (5) auf einen Druck unterhalb des Umgebungsdruckes.

52. Vorrichtung nach einem der Ansprüche 46 bis 51, **dadurch gekennzeichnet,** daß die dichten Wände vergleichsweise undehnbar in der Umfangszone und vergleichsweise elastisch dehnbar in der oberen Zone sind.

53. Vorrichtung nach einem der Ansprüche 46 bis 52 in Verbindung mit Anspruch 11, **dadurch gekennzeichnet,** daß der besagte Überzug vergleichsweise undehnbar in einer der Umfangszone entsprechenden Zone und vergleichsweise elastisch dehnbar in einer der oberen Zone entsprechenden Zone ist.

54. Vorrichtung nach einem der Ansprüche 1 bis 40, **dadurch gekennzeichnet,** er aus einer Vorrichtung (126, 127, 173) zur Einstellung und/oder Unterstützung mindestens eines Objektes (132, 170) in einem Hohlraum (124, 125, 167) mit einer Innenkontur, wie einem Gepäckstück, einem Behälter, einem Fahrzeugkofferraum oder dergleichen besteht, wobei die besagte starre, einhüllende Schale (1) gegenüber von dem besagten inneren Volumen (4) sich an die besagte Innenkontur anschmiegt.
